(19) Europäisches Patentamt
European Patent Office
Office européen des brevets

(11) Publication number : **0 628 551 A1**

(12) # EUROPEAN PATENT APPLICATION

(21) Application number : **94304289.5**

(22) Date of filing : **14.06.94**

(51) Int. Cl.[5] : **C07D 295/20,** C07D 295/22, C07D 207/26, C07D 211/14, C07D 211/22, C07D 211/46, A61K 31/445, A61K 31/50, A61K 31/54, C07D 211/70, C07D 213/74, C07D 239/42, C07D 241/20, C07D 279/12, C07D 401/04, C07D 401/12, C07D 417/12, C07D 417/14

(30) Priority : **14.06.93 JP 142361/93**
**06.06.94 JP 124087/94**

(43) Date of publication of application :
**14.12.94 Bulletin 94/50**

(84) Designated Contracting States :
**AT BE CH DE DK ES FR GB GR IE IT LI LU MC NL PT SE**

(71) Applicant : **SHIONOGI & CO., LTD.**
**1-8, Doshomachi 3-chome,**
**Chuo-ku**
**Osaka-shi, Osaka-fu (JP)**

(72) Inventor : **Matsumura, Hiromu**
**15-10-839, Asahigaoka-cho**
**Ashiya-shi, Hyogo-ken (JP)**
Inventor : **Yano, Toshisada**
**5-19-18, Kita-ochiai,**
**Suma-ku**
**Kobe-shi, Hyogo-ken (JP)**
Inventor : **Hashizume, Hiroshi**
**1-1-1118, Ikeda-cho,**
**Kita-ku**
**Osaka-shi, Osaka (JP)**
Inventor : **Eigyou, Masami**
**2-10-7, Shikanodai-nishi**
**Ikoma-shi, Nara-ken (JP)**
Inventor : **Ohtani, Kohichi**
**5-8, Daishi-cho**
**Kawachinagano-shi, Osaka (JP)**
Inventor : **Arimura, Akinori**
**1-14-22-201, Abiko-nishi,**
**Sumiyoshi-ku**
**Osaka-shi, Osaka (JP)**

(74) Representative : **Nash, David Allan et al**
**Haseltine Lake & Co.**
**Hazlitt House**
**28 Southampton Buildings**
**Chancery Lane**
**London WC2A 1AT (GB)**

(54) **Semicyclic urea derivatives as antihistaminic agents.**

(57) A compound represented by the following Formula I :

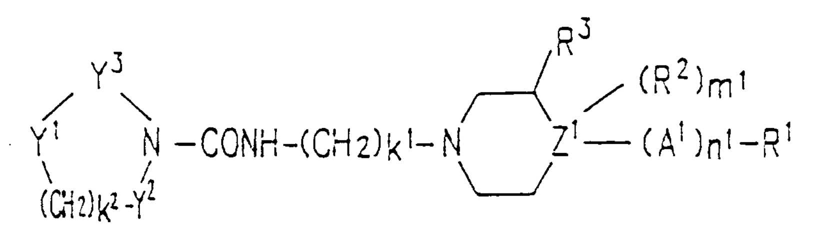

(I)

wherein $Z^1$ is C or N ; $m^1$ is 0 when $Z^1$ is N and $m^1$ is 1 when $Z^1$ is C ; $R^3$ can form a bond with $R^2$ when $Z^1$ is C ; $A^1$ is O, $SO_2$, or $CH_2$ ; $n^1$ is 0 or 1 when $A^1$ is O or $SO_2$ and $n^1$ is an integer of 0 to 3 when $A^1$ is $CH_2$ ; $R^1$ is a group selected from the group consisting of a condensed aromatic ring, a substituted condensed aromatic ring, carboxyl, alkoxycarbonyl, and

EP 0 628 551 A1

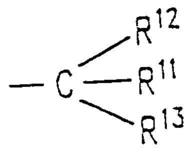

wherein $R^{11}$ is H or OH, or $R^{11}$ can form a bond with $R^2$ when $Z^1$ is C and $n^1$ is O ; $R^{12}$ and $R^{13}$ are independently phenyl, substituted phenyl, a heterocyclic ring, or a substituted heterocyclic ring, or $R^{12}$ and $R^{13}$ can form a condensed ring ; $R^2$ is H when forming no bond with $R^3$ or $R^{11}$ ; $R^3$ is H when forming no bond with $R^2$ ; $k^1$ is an integer of 2 to 5 ; $Y^1$ is a group selected from the group consisting of O, S, SO, $SO_2$, $CH_2$, and

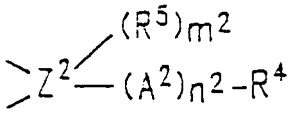

wherein $Z^2$ is N or C ; $m^2$ is 0 when $Z^2$ is N and $m^2$ is 1 when $Z^2$ is C ; $A^2$ is O, $SO_2$, or $CH_2$ ; $n^2$ is 0 or 1 when $A^2$ is O or $SO_2$ ; $n^2$ is an integer of 0 to 3 when $A^2$ is $CH_2$ ; $R^4$ is a group selected from the group consisting of alkyl, phenyl, substituted phenyl, a heterocyclic ring, a substituted heterocyclic ring, $-CO-R^{41}$, and

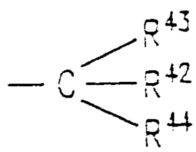

wherein $R^{41}$ is a group selected from the group consisting of OH, alkoxy, amino, arylalkyloxy, substituted amino, arylalkenyl, and substituted arylalkenyl ; $R^{42}$ is H or OH, or $R^{42}$ can form a bond with $R^5$ when $Z^2$ is C and $n^2$ is 0 ; $R^{43}$ and $R^{44}$ are independently phenyl, substituted phenyl, a heterocyclic ring, or a substituted heterocyclic ring ; $R^5$ is H when forming no bond with $R^{42}$ ; $Y^2$ is $CH_2$ or CO ; $Y^3$ is $(CH_2)_2$ or phenylene ; and $k^2$ is 0 or 1, or pharmaceutically acceptable salts thereof.

## BACKGROUND OF THE INVENTION

1. Field of the Invention:

The present invention relates to a novel urea derivative containing a heterocyclic ring, and more specifically to a novel compound having an antihistaminic effect, useful as an antiallergic agent, and antiallergic agents containing the same.

2. Description of the Related Art:

In recent years, various kinds of compounds have been known as antihistaminic antiallergic agents. Examples of the compounds include thiazole derivatives disclosed in Japanese Laid-Open Patent Publication No. 63-225374, piperidine derivatives disclosed in Japanese Patent Publication No. 4-32821, phenyl derivatives disclosed in Japanese Patent Publication No. 2-28566, and piperazine derivatives disclosed in Japanese Laid-Open Patent Publication No. 63-188670.

Currently used antihistaminic agents useful as the above-mentioned antiallergic agents have strong antihistaminic effects, but involve some side effects on the central nervous system, such as sleepiness and ataxy. Considering these undesirable side effects, improved antiallergic agents have been developed. In general, however, new compounds which alleviate undesirable side effects also have decreased antihistaminic effects. Thus, there is a need for compounds having high antihistaminic and antiallergic effects which do not have undesirable side effects.

## SUMMARY OF THE INVENTION

The compound of this invention is represented by the following Formula I.

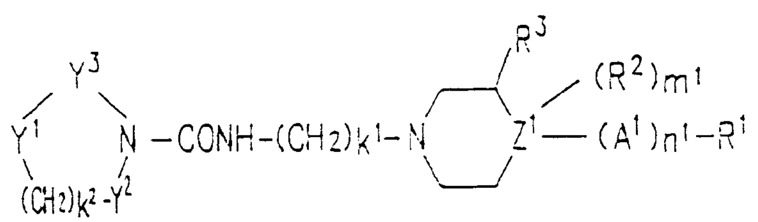

(I)

wherein $Z^1$ is C or N; $m^1$ is 0 when $Z^1$ is N and $m^1$ is 1 when $Z^1$ is C; $R^3$ can form a bond with $R^2$ when $Z^1$ is C; $A^1$ is O, $SO_2$, or $CH_2$; $n^1$ is 0 or 1 when $A^1$ is O or $SO_2$ and $n^1$ is an integer of 0 to 3 when $A^1$ is $CH_2$; $R^1$ is a group selected from the group consisting of a condensed aromatic ring, a substituted condensed aromatic ring, carboxyl, alkoxycarbonyl, and

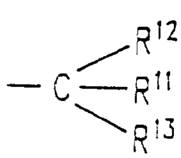

wherein $R^{11}$ is H or OH, or $R^{11}$ can form a bond with $R^2$ when $Z^1$ is C and $n^1$ is 0; $R^{12}$ and $R^{13}$ are independently phenyl, substituted phenyl, a heterocyclic ring, or a substituted heterocyclic ring, or $R^{12}$ and $R^{13}$ can form a condensed ring; $R^2$ is H when forming no bond with $R^3$ or $R^{11}$; $R^3$ is H when forming no bond with $R^2$; $k^1$ is an integer of 2 to 5; $Y^1$ is a group selected from the group consisting of O, S, SO, $SO_2$, $CH_2$, and

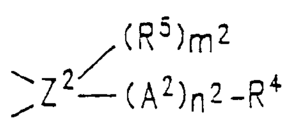

wherein $Z^2$ is N or C; $m^2$ is 0 when $Z^2$ is N and $m^2$ is 1 when $z^2$ is C; $A^2$ is O, $SO_2$, or $CH_2$; $n^2$ is 0 or 1 when $A^2$ is O or $SO_2$; $n^2$ is an integer of 0 to 3 when $A^2$ is $CH_2$; $R^4$ is a group selected from the group consisting of alkyl, phenyl, substituted phenyl, a heterocyclic ring, a substituted heterocyclic ring, -CO-$R^{41}$, and

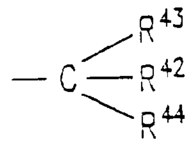

wherein $R^{41}$ is a group selected from the group consisting of OH, alkoxy, amino, arylalkyloxy, substituted amino, arylalkenyl, and substituted arylalkenyl; $R^{42}$ is H or OH, or $R^{42}$ can form a bond with $R^5$ when $Z^2$ is C and $n^2$ is 0; $R^{43}$ and $R^{44}$ are independently phenyl, substituted phenyl, a heterocyclic ring, or a substituted heterocyclic ring; $R^5$ is H when forming no bond with $R^{42}$; $Y^2$ is $CH_2$ or CO; $Y^3$ is $(CH_2)_2$ or phenylene; and $k^2$ is 0 or 1.

The present invention also includes pharmaceutically acceptable salts of the above-mentioned compounds.

In one embodiment, the compound is represented by the following Formula I-1:

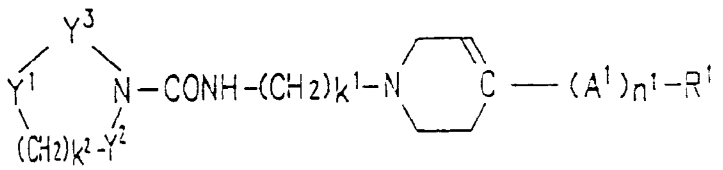

(I-1)

wherein $A^1$, $R^1$, $Y^1$, $Y^2$, $Y^3$, $k^1$, $k^2$, and $n^1$ are the same as defined above.

In one embodiment, $R^1$ in Formula I is quinolyl or

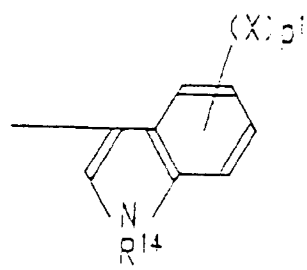

wherein $R^{14}$ is H, phenyl, or substituted phenyl; $p^1$ is an integer of 0 to 4; and X's are independently a group selected from the group consisting of Cl, F, $CH_3$, CN, and $CH_2COOH$.

In one embodiment, the compound is represented by the following Formula I-2:

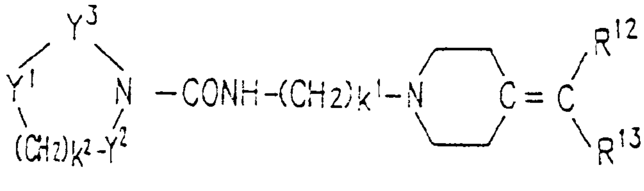

(I-2)

wherein $R^{12}$, $R^{13}$, $Y^1$, $Y^2$, $Y^3$, $k^1$, and $k^2$ are the same as defined above.

In one embodiment, $R^1$ in Formula I is

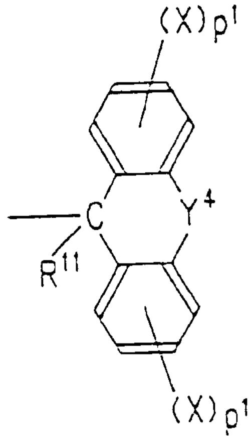

wherein $Y^4$ is $(CH_2)k^3$ or $CH_2$-$Y^5$, where $k^3$ is an integer of 0 to 2, $Y^5$ is O, S, or $NR^5$, where $R^5$ is H or $CH_3$; $p^1$ is an integer of 0 to 4; and X's are independently a group selected from the group consisting of Cl, F, $CH_3$, CN, and $CH_2COOH$.

In one embodiment, $R^4$ in Formula I is a group selected from the group consisting of pyridyl, pyrimidinyl, imidazolyl, quinolyl, and

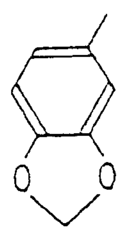

In one embodiment, the compound is represented by the following Formula I-3:

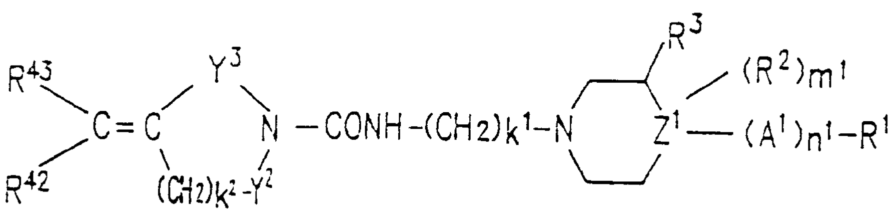
(I-3)

wherein $A^1$, $R^1$, $R^2$, $R^3$, $R^{43}$, $R^{42}$, $Y^2$, $Y^3$, $Z^1$, $k^1$, $k^2$, $m^1$, and $n^1$ are the same as defined above.

In one embodiment, the compound is represented by the following Formula I-4:

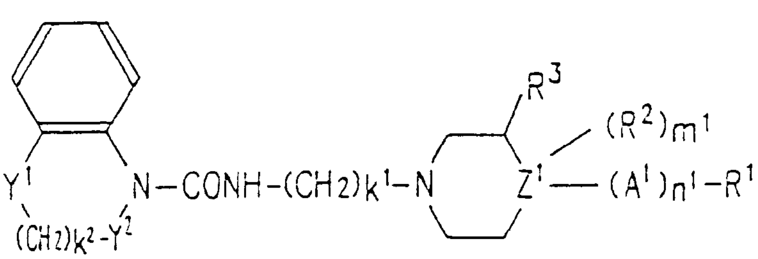
(I-4)

wherein $A^1$, $R^1$, $R^2$, $R^3$, $Y^1$, $Y^2$, $Z^1$, $k^1$, $k^2$, $m^1$, and $n^1$ are the same as defined above.

In one embodiment, in Formula I, $A^1$ is $CH_2$ and $R^1$ is carboxyl or alkoxycarbonyl.

In one embodiment, in Formula I, $Z^1$ is N and $n^1$ is 0.

In one embodiment, in Formula I, $Z^1$ is C, $A^1$ is O, and $n^1$ is 1.

In one embodiment, in Formula I, $y^2$ is $CH_2$, $Y^3$ is $(CH_2)_2$, and $K^2$ is 1.

In one embodiment, in Formula I, $Y^1$ is S or $CH_2$, $y^2$ is CO, and $Y^3$ is $(CH_2)_2$.

In one embodiment, in Formula I, $R^1$ is represented by the following Formula:

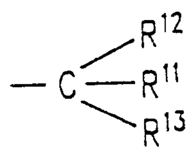

wherein $R^{11}$, $R^{12}$ and $R^{13}$ are the same as defined above.

The antiallergic agent of the present invention comprises an effective amount of the above-mentioned compounds or pharmaceutically acceptable salts thereof.

The composition of the present invention comprises the compound of claim 1 or pharmaceutically acceptable salts thereof and a carrier.

Thus, the invention described herein makes possible the advantages of (1) providing a novel compound having strong antihistaminic effects; (2) providing a novel compound having high affinity for a histamine receptor; (3) providing a novel compound having strong antihistaminic effects and having a low degree of migration to brain when the compound is administered; (4) providing a novel compound having a 5-lipoxygenase inhibiting effect; (5) providing a novel compound having inhibiting effects on various chemical mediators; and (6) providing antiallergic agents having excellent antihistaminic effects and decreased side effects.

These and other advantages of the present invention will become apparent to those skilled in the art upon reading and understanding the following detailed description.

## DESCRIPTION OF THE PREFERRED EMBODIMENTS

The compound of the present invention is a compound represented by the following general Formula I:

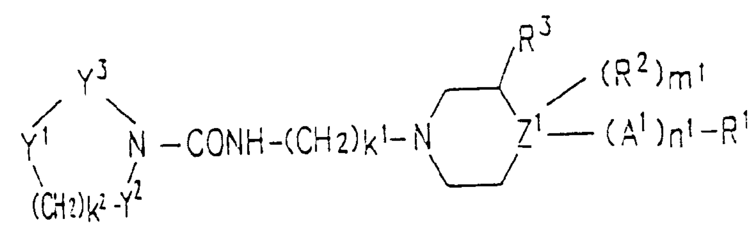 (I)

wherein $Z^1$ is C or N; $m^1$ is 0 when $Z^1$ is N and $m^1$ is 1 when $Z^1$ is C; $R^3$ can form a bond with $R^2$ when $Z^1$ is C; $A^1$ is O, $SO_2$, or $CH_2$; $n^1$ is 0 or 1 when $A^1$ is O or $SO_2$ and $n^1$ is an integer of 0 to 3 when $A^1$ is $CH_2$; $R^1$ is a group selected from the group consisting of a condensed aromatic ring, a substituted condensed aromatic ring, carboxyl, alkoxycarbonyl, and

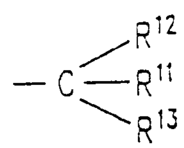

wherein $R^{11}$ is H or OH, or $R^{11}$ can form a bond with $R^2$ when $Z^1$ is C and $n^1$ is 0; $R^{12}$ and $R^{13}$ are independently phenyl, substituted phenyl, a heterocyclic ring, or a substituted heterocyclic ring, or $R^{12}$ and $R^{13}$ can form a condensed ring; $R^2$ is H when forming no bond with $R^3$ or $R^{11}$; $R^3$ is H when forming no bond with $R^2$; $k^1$ is an integer of 2 to 5; $Y^1$ is a group selected from the group consisting of O, S, SO, $SO_2$, $CH_2$, and

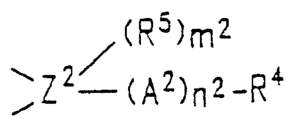

wherein $Z^2$ is N or C; $m^2$ is 0 when $Z^2$ is N and $m^2$ is 1 when $Z^2$ is C; $A^2$ is O, $SO_2$, or $CH_2$; $n^2$ is 0 or 1 when $A^2$ is O or $SO_2$; $n^2$ is an integer of 0 to 3 when $A^2$ is $CH_2$; $R^4$ is a group selected from the group consisting of alkyl, phenyl, substituted phenyl, a heterocyclic ring, a substituted heterocyclic ring, -CO-$R^{41}$, and

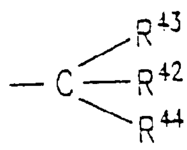

wherein $R^{41}$ is a group selected from the group consisting of OH, alkoxy, amino, arylalkyloxy, substituted amino, arylalkenyl, and substituted arylalkenyl; $R^{42}$ is H or OH, or $R^{42}$ can form a bond with $R^5$ when $Z^2$ is C and $n^2$ = 0; $R^{43}$ and $R^{44}$ are independently phenyl, substituted phenyl, a heterocyclic ring, or a substituted heterocyclic ring; $R^5$ is H when forming no bond with $R^{42}$; $Y^2$ is $CH_2$ or CO; $Y^3$ is $(CH_2)_2$ or phenylene; and $k^2$ is 0 or 1.

In the above-mentioned Formula I, $R^3$ can form a bond with $R^2$ when $Z^1$ is C. Herein, the formation of a bond between $R^2$ and $R^3$ means that $Z^1$ is double-bonded to its adjacent carbon constituting a ring. Thus, in that case, the compound of Formula I can be represented by the following Formula I-1:

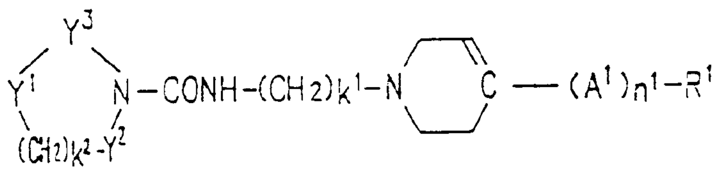

(I-1)

where $A^1$, $R^1$, $Y^1$, $Y^2$, $Y^3$, $k^1$, $k^2$, and $n^1$ are the same as defined in Formula I.

When $R^1$ is a condensed aromatic ring, the condensed aromatic ring can contain at least one substituent thereon, where the condensed aromatic ring includes a condensed aromatic heterocyclic ring. Examples of the substituent include halogen, alkyl, aryl, cyano, carboxyalkyl, amino, and the like. Examples of the halogen include fluorine, chlorine, bromine, and iodine. The alkyl and alkyl in the carboxyalkyl refer to a straight-chain or branched-chain alkyl group having 1 to 5 carbon atoms. Examples of the alkyl group include methyl, ethyl, n-propyl, isopropyl, n-butyl, isobutyl, sec-butyl, tert-butyl, n-pentyl, isopentyl, neopentyl, and the like. Examples of the aryl include phenyl, naphthyl, and the like, and the phenyl is preferred. The alkyl or aryl can be substituted by a substituent such as halogen. The substituent on the above-mentioned condensed aromatic ring is preferably selected from the group consisting of Cl, F, $CH_3$, CN, halogenated phenyl, and $CH_2COOH$. The condensed aromatic ring is preferably quinolyl or

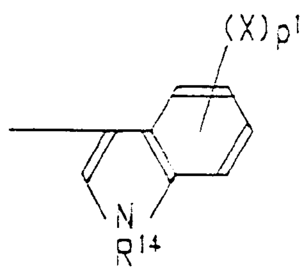

where $R^{14}$ is H, phenyl, or substituted phenyl; $p^1$ is an integer of 0 to 4; X's are independently a group selected from the group consisting of Cl, F, $CH_3$, CN, and $CH_2COOH$.

In the above Formula I, the alkoxy carbonyl represented by $R^1$ is a carbonyl group substituted by alkoxy. The alkoxy has 1 to 5 carbon atoms. Examples of the alkoxy include methoxy, ethoxy, propoxy, isopropoxy, butoxy, and the like.

$A^1$ is preferably $CH_2$ when $R^1$ is carboxyl or alkoxycarbonyl.

$R^{11}$ can form a bond with $R^2$, when $Z^1$ is C and $n^1$ = 0. Herein, the formation of a bond between $R^2$ and $R^{11}$ means that $R^1$ is double-bonded to $Z^1$. More specifically, this indicates that the compound of the present invention is represented by the following Formula I-2:

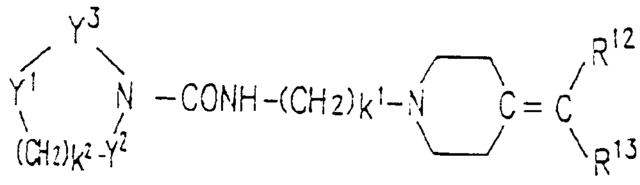 (I-2)

where $R^{12}$, $R^{13}$, $Y^1$, $Y^2$, $y^3$, $k^1$, and $k^2$ are the same as defined in Formula I.

The substituted phenyl groups represented by $R^{12}$ and $R^{13}$ have at least one substituent thereon, independently. Examples of the substituents include the same substituents as those on the condensed aromatic ring represented by $R^1$. The substituents on the phenyl groups are preferably selected from the group consisting of Cl, F, $CH_3$, CN, and $CH_2COOH$.

The substituted heterocyclic rings represented by $R^{12}$ and $R^{13}$ have at least one substituent thereon. Herein, the heterocyclic ring refers to five or six membered ring containing nitrogen, oxygen and/or sulfur as a heteroatom. Examples of the heterocyclic ring include furyl, thienyl, pyrimidinyl, pyridyl, isoxazolyl, thiazolyl. Pyridyl is preferred. Examples of the substituent on the heterocyclic ring include the same substituents as those on the condensed aromatic ring represented by $R^1$.

It is preferred that both $R^{12}$ and $R^{13}$ are phenyl groups or substituted phenyl groups or at least one of $R^{12}$ and $R^{13}$ is pyridyl.

$R^{12}$ and $R^{13}$ can form a condensed ring. Herein, the formation of a condensed ring by $R^{12}$ and $R^{13}$ means that carbon atoms of $R^{12}$ and $R^{13}$ constitute carbon atoms of a condensed ring. This condensed ring may have at least one substituent thereron. Examples of the substituent on the condensed ring can be the same substituents as those on the condensed aromatic ring represented by $R^1$. In the case where $R^{12}$ and $R^{13}$ form a condensed ring, $R^1$ is preferably a group represented by the following Formula:

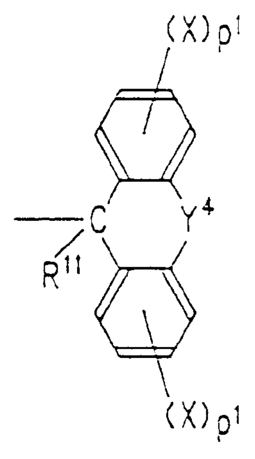

where $Y^4$ is $(CH_2)k^3$ or $CH_2$-$Y^5$, wherein $k^3$ is an integer of 0 to 2 and $y^5$ is O, S, or $NR^5$, wherein $R^5$ is H or $CH_3$; $p^1$'s are independently integers of 0 to 4; X's are independently a group selected from the group consisting of Cl, F, $CH_3$, CN, and $CH_2COOH$.

In the above Formula I, when $R^4$ is alkyl, the alkyl is a straight-chain or branched-chain alkyl group having 1 to 5 carbon atoms. Examples of the alkyl group include methyl, ethyl, n-propyl, isopropyl, n-butyl, isobutyl, sec-butyl, tert-butyl, n-pentyl, isopentyl, and neopentyl. Methyl is preferred.

The substituted phenyl represented by $R^4$ have at least one substituent thereon. Examples of the substituent include the same substituents as those on the condensed aromatic ring represented by $R^1$. The substituent on the phenyl is preferably alkyl or halogen.

The above-mentioned substituted heterocyclic ring represented by $R^4$ has at least one substituent thereon. Herein, the heterocyclic ring refers to a cyclic group containing nitrogen, oxygen, and/or sulfur as a heteroatom, and the heterocyclic ring can be a condensed ring. Examples of the heterocyclic ring include five or six membered rings such as furyl, thienyl, pyrimidinyl, pyridyl, and imidazolyl or condensed heterocyclic rings such as quinolyl and

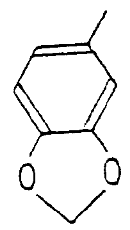

Examples of the substituent on the heterocyclic ring include the same substituents as those on the condensed aromatic ring represented by $R^1$.

When $R^4$ is $COR^{41}$ and $R^{41}$ is alkoxy, $R^{41}$ has 1 to 5 carbon atoms. Examples of alkoxy include methoxy, ethoxy, propoxy, isopropoxy, butoxy, tert-butoxy, pentyloxy, and the like.

The arylalkyloxy represented by $R^{41}$ is an alkyloxy group substituted by aryl. Examples of the aryl include phenyl and the like. An alkyl in the alkyloxy has 1 to 5 carbon atoms, and methyl is preferred. Preferably, the arylalkyloxy represented $R^{41}$ is benzhydryl.

The above-mentioned substituted amino group represented by $R^{41}$ has 1 or 2 substituents. Examples of the substituent include alkyl, and the like. The alkyl is the same as defined in the alkyl represented by $R^4$ and is preferably methyl. The amino group is preferably substituted by two substituents.

The above-mentioned arylalkenyl represented by $R^{41}$ is an alkenyl group substituted by aryl. Examples of the aryl group include phenyl, and the like. The alkenyl group has 2 to 3 carbon atoms. Examples of the alkenyl group include ethenyl, propenyl, and the like. The arylalkenyl represented by $R^{41}$ is preferably phenylethenyl.

The above-mentioned substituted arylalkenyl represented by $R^{41}$ is an alkenyl group substituted by aryl. The aryl group is substituted by at least one substituent. Examples of the aryl group and the alkenyl group inclues the same groups as those on the arylalkenyl represented by $R^{41}$. Examples of the substituent on the aryl group include alkoxy, alkoxycarbonyloxy, and the like. The substitueted arylalkenyl represented by $R^{41}$ is preferably 4-ethoxycarbonyloxy-3-methoxyphenylethenyl.

$R^{42}$ can form a bond with $R^5$ when $Z^2$ is C and $n^5 = 0$. The formation of a bond by $R^{42}$ and $R^5$ means that $R^4$ is double-bonded to $Z^2$. More specifically, this indicates that the compound of the present invention is represented by the following Formula I-3:

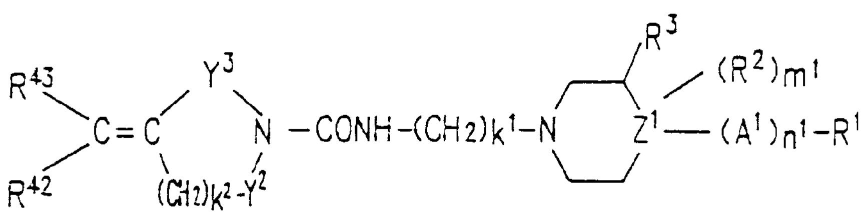

(I-3)

where $A^1$, $R^1$, $R^2$, $R^3$, $R^{42}$, $R^{43}$, $Y^2$, $Y^3$, $Z^1$, $k^1$, $k^2$, $m^1$ and $n^1$ are the same as defined in Formula I.

The above-mentioned substituted phenyl group and substituted heterocyclic ring represented by $R^{43}$ and $R^{44}$, respectively are the same as defined in the substituted phenyl group and substituted heterocyclic ring represented by $R^{12}$ and $R^{13}$.

$Y^3$ can be phenylene. This phenylene is preferably o-phenylene. In the case where $Y^3$ is o-phenylene, the compound of the present invention can be represented by the followina Formula I-4:

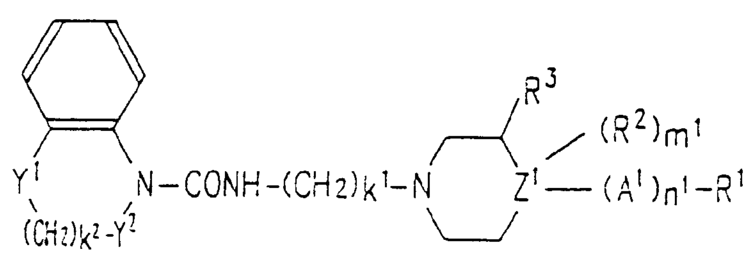

(I-4)

The compound of the present invention can be a pharmaceutically acceptable salt of the compound represented by Formula I.

Examples of the pharmaceutically acceptable salts include appropriate organic or inorganic acid salts such

as oxalate, maleate, tartarate, phosphate, acetate, benzoate, hydrochloride, nitrate, and hydroiodide.

The salts can be readly formed, for example, by the addition of a solution of a desired acid or its salt to a sloution of the compound represented by Formula I.

The compound represented by Formula I can be obtained, for example, by effecting the following reaction:

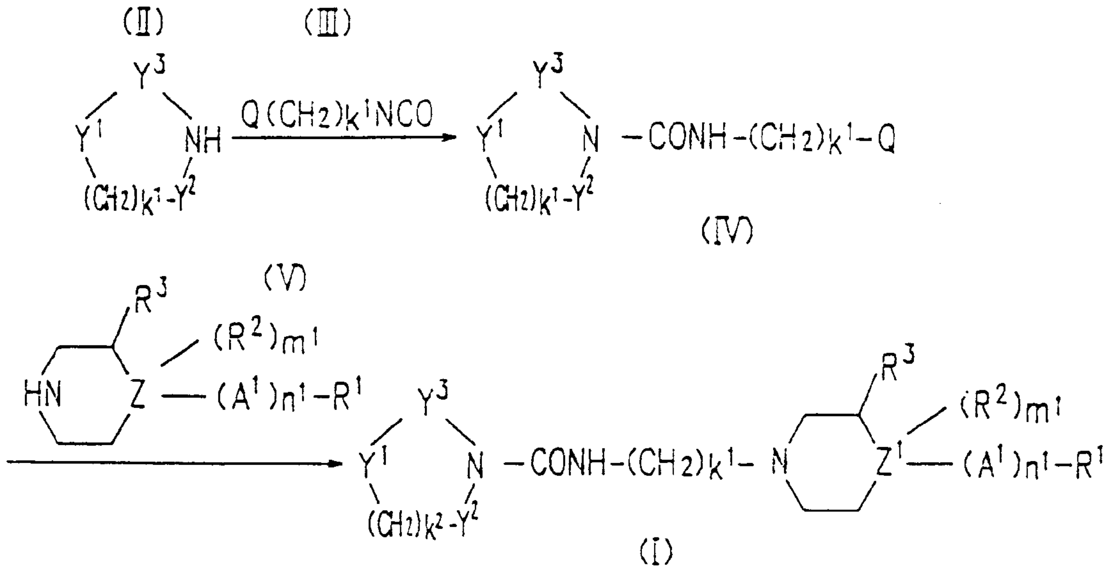


where $A^1$, $R^1$, $R^2$, $R^3$, $Y^1$, $Y^2$, $Y^3$, $k^1$, $k^2$, $m^1$, and $n^1$ are the same as defined in Formula I; Q is halogen such as chlorine, bromine, and iodine.

In this method, Amine II is allowed to react with Isocyanate III in the presence of tertiary amine such as triethylamine, and if required, an organic solvent such as dichloromethane, chloroform, and acetonitrile at a temperature of 0 to 150°C to obtain Urea derivative IV. Urea derivative IV is allowed to react with Amine V to obtain Compound I of the present invention. The reaction between Urea derivative IV and Amine V is effected in an appropriate solvent at a temperature of 10 to 120°C, and more preferably 50 to 100°C. Examples of the solvent include acetonitrile, benzene, dimethyl sulfoxide, dimethyl formamide, chloroform, ethyl acetate, and tetrahydrofuran. If required, a base can be added as a neutralizing agent. Examples of the base include organic bases such as pyridine, triethylamine, and dimethylaniline or inorganic bases such as $K_2CO_3$, NaH, and NaOH. In order to further allow the reaction to proceed, alkali metal iodide such as NaI and KI can be used.

The compound represented by Formula I-2 in which $R^1$ is double-bonded to $Z^1$ can be obtained, for example by effecting the following reaction:

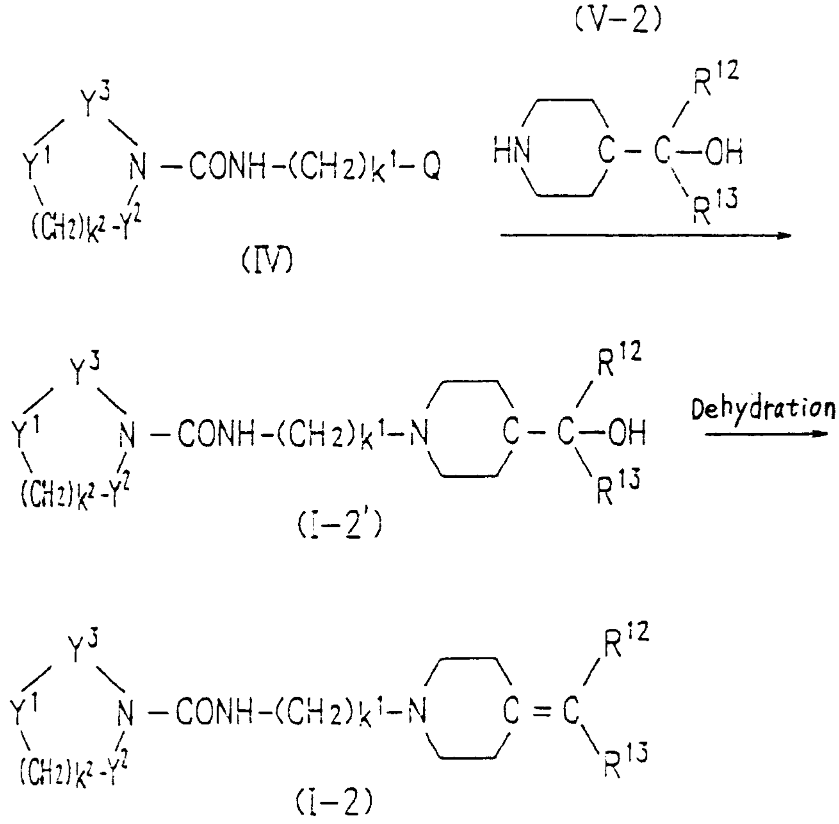


(V-2)

(IV)

(I-2')

(I-2)

where $R^{12}$, $R^{13}$, $Y^1$, $Y^2$, $Y^3$, $k^1$, and $k^2$ are the same as defined in Formula I; and Q is halogen such as chlorine, bromine, and iodine.

In this method, Urea derivative IV is first allowed to react with Amine V-2 having a hydroxyl group to obtain Compound I-2' of the present invention. The conditions for this reaction are the same as those used in the above-mentioned method. The resulting Compound I-2' is dehydrated to obtain Compound I-2 having a double bond of the present invention. This dehydration can be effected by treating Compound I-2' in an organic solvent in the presence of acid such as $CF_3COOH$, hydrochloric acid, acetic acid, and p-toluenesulfonic acid at a temperature of 0 to 120°C. Examples of the organic solvent include methanol, ethanol, tetrahydrofuran, dimethylformamide, acetonitrile, benzene, toluene, and diethyl ether.

The effects of Compound I of the present invention and salts thereof as an antiallergic agents can be evaluated by various methods for measuring antiallergic activity, for example by conducting the following tests (1) and (2).

(1) Antihistaminic effect evaluation test in vitro

Histamine diphosphate and Compound I of the present invention or salts thereof are added to an ileum of a guinea pig in a saline solution. The concentration (at which the contraction of the ileum by histamine is inhibited by 50%) of Compound I of the present invention is measured.

(2) $Histamine_1$ receptor ($H_1$ receptor) in vitro binding inhibition test

As a standard material which binds to a $H_1$ receptor, pyrilamine is used. Compound I of the present invention is added with varying concentrations to a mixture containing a receptor sample prepared by homogenizing a cerebral cortex of a rat and [$^3$H]-pyrilamine, and incubated. From radioactivity of [$^3$H]-pyrilamine which is bound to the receptor sample, the concentration ($IC_{50}$) of Compound I inhibiting the specific binding of [$^3$H]-pyrilamine to the receptor sample is measured to obtain an inhibition constant (Ki).

Compound I of the present invention has a lower degree of migration to brain. Thus, Compound I has less side effects on the central nervous system such as sleepiness, because of its less frequent binding to the $H_1$ recepter in brain. The extent of migration of Compound I or salts thereof to the brain can be obtained by the following test method 3.

(3) $H_1$ receptor binding inhibition test ex vivo

A cerebral cortex of a mouse orally administered with Compound I of the present invention is homogenized to prepare a receptor sample. The receptor sample is incubated with [$^3$H]-pyrilamine. From the radioactivity of [$^3$H]-pyrilamine bound to the receptor sample, the inhibition of Compound I with respect to the specific biding of pyrilamine to the receptor sample is obtained.

As apparent from examples described later, Compound I of the present invention exhibits antihistaminic effects which are more excellent than those of known compounds such as terfenadine and oxatomide, and its migration to a brain is lower. This reveals that Compound I of the present invention and salts thereof can be used as an antihistaminic agent and can be applied to the prevention and treatment of allergic diseases such as bronchial asthma, allergic rhinitis, urticaria, atopic dermatitis, and eczema, involving less side effects.

Furthermore, Compound I of the present invention exhibits a repressing effect such as an excellent antagonistic effect on various chemical mediators in addition to histamine. For example, some of the embodiments of Compound I of the present invention have excellent 5-lipoxygenase inhibiting effects, and hence such compounds have effects such as anti-inflammatory effects.

The administration of a pharmaceutical composition containing Compound I of the present invention or salts thereof can be conducted by oral administration, parenteral administration, intrarectal administration, or topical administration. Oral administration or topical administration is preferred. The dose of Compound I or salts thereof is varied depending upon the specific embodiment of the compound, administration method, conditions and age of a patient. Typically, 0.005 to 40 mg/kg/day, and preferably 0.01 to 5 mg/kg/day are used. In general, Compound I or salts thereof can be administered in the form of a formulation prepared by mixing Compound I or salts thereof with a carrier for a formulation. As the carrier for a formulation, materials satisfying the following conditions are used: materials which are widely in use in the formulation field and do not react with the compound of the present invention and salts thereof. Examples of the carrier include lactose, glucose, mannitol, dextrin, cyclodextrin, starch, saccharose, magnesium aluminate metasilicate, synthetic aluminum silicate, crystal cellulose, sodium carboxymethyl cellulose, hydroxypropyl starch, calcium caroboxymethyl cellulose, ionic exchange resin, methylcellulose, gelatin, gum arabic, hydroxypropyl cellulose, hydroxypropyl cellulose with low substitution degree, hydroxypropylmethyl cellulose, polyvinylpyrrolidone, polyvinyl alcohol, light silicic anhydride, magnesium stearate, talc, tragacanth gum, bentonite, beeswax, carboxyvinyl polymer, titanium oxide, sorbitan-aliphatic acid ester, sodium lauryl sulfate, glycerin, aliphatic acid-glycerin ester, purified lanolin, glycerogelatin, polysolbate, macrogol, vegetable oil, wax, liquid paraffin, white vaseline, fluorocarbon, nonionic surfactant, propylene glycol, and water. Examples of the formulation type include tablets, capsules, granules, powders, syrup, suspension, suppository, ointment, cream, gel, patch, inhalation, and injection. These formulations are prepared by the conventional methods. Liquid formulations can be prepared so as to be dissolved or suspended in water or other appropriate solvents when used. Tablets and granules can be coated by known method. Injection can be prepared by dissolving a pharmaceutically acceptable salt of Compound I in water. If required, the salt can be dissolved in a saline or in a glucose solution, and a buffer or preservatives can be further added. These formulations can contain Compound I or salts thereof in an amount of 0.2% or more, and preferably 0.5 to 70%. These formulations can further contain other therapeutically effective components.

## Examples

Hereinafter, the present invention will be described by way of illustrative examples and reference examples.

## Reference Example 1

Synthesis of 4-(3-chloropropylcarbamoyl)-3-oxothiomorpholine as an intermediate

The synthesis was conducted according to the following reaction scheme.

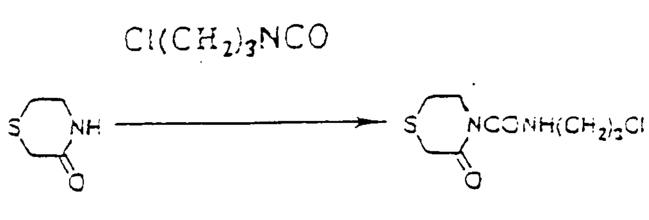


First, a mixture containing 14 g (119 mmol) of 3-oxothiomorpholine, 15 g (125 mmol) of chloropropyl isocyanate, and 0.5 ml of triethylamine was heated with stirring at 100°C for 9 hours. The reaction mixture was cooled. Then, the mixture thus obtained was purified by silica gel column chromatography using toluene-ethyl acetate (15/1 to 10/1 v/v) as an eluent to obtain 27 g of an oily product (yield: 96%).

IR($CHCl_3$)($cm^{-1}$) 3300, 3001, 2950, 1710, 1668, 1540

NMR($CDCl_3$) 2.053(quint. j=7Hz, 2H), 2.985(t, j=6Hz, 2H), 3.363(s, 2H), 3.487(q, j=7Hz, 2H), 3.602(t, j=6Hz,

2H), 4.214(t, j=6Hz, 2H), 9.05 to 9.03(m, 1H)

Example 1

Synthesis of 3-{4-(diphenylhydroxymethyl)-piperidin-1-yl)-propylcarbamoyl}-3-oxothiomorpholine(Ia') and its oxalate (Ia)

The synthesis was conducted according to the following reaction scheme.

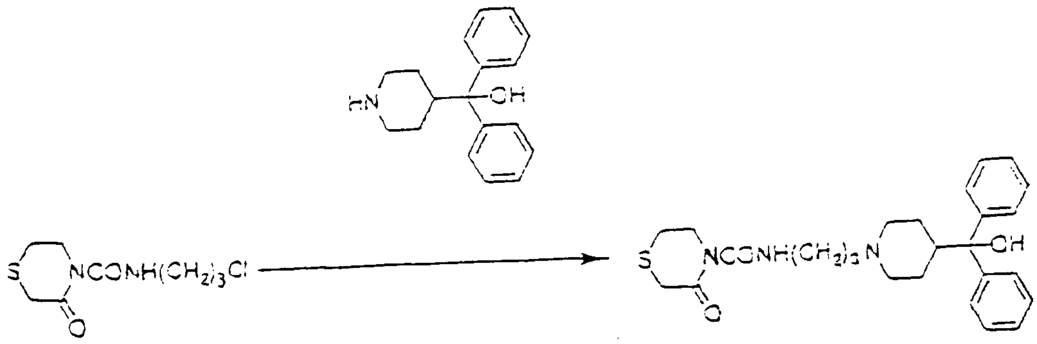

(Ia')

First, 3.08 g (13 mmol) of 4-(3-chloropropylcarbamoyl)-3-oxothiomorpholine obtained in Reference Example 1 and 3.48 g (13 mmol) of α,α-diphenyl-4-piperidinomethanol were dissolved in 30 ml of acetonitrile. To this reaction mixture, 3.59 g (26 mmol) of $K_2CO_3$ and 1.08 g (6.5 mmol) of KI were added and heated with stirring at 90°C for 17 hours. Methylene chloride was added to the resulting mixture, washed, and dried, and the solvent was removed under reduced pressure. The residue was purified by silica gel column chromatography using dichloromethaneisopropanol (10/1 v/v) as an eluent to obtain 3.9 g of an oily product Ia' (yield: 64%).

IR($CHCl_3$)($cm^{-1}$) 3607, 3299, 3058, 2949, 2811, 2741, 1706, 1665, 1599, 1540

NMR($CDCl_3$)1.40 to 1.75(m, 4H), 1.784(quint, j=7Hz, 2H), 1.95 to 2.15(m, 2H), 2.20 to 2.50(m, 1H), 2.481(t, j=7Hz, 2H), 2.55 to 2.70(m, 1H), 2.962(t, j=6Hz, 2H), 2.90 to 3.10(m, 2H), 3.360(s, 2H), 3.368(q, j=7Hz, 2H), 4.191(t, j=6Hz, 2H), 7.10 to 7.55(m, 10H), 9.10 to 9.30(m, 1H)

Product Ia' was recrystallized from methanolisopropanol with conversion to an oxalate to obtain a purified product Ia as crystals having a melting point (d) of 161.0 to 162.5°C.

| Elem. Anal. for $C_{26}H_{33}N_3O_3S \cdot 1.5C_2H_2O_4$ FW 602.688 | | | | |
|---|---|---|---|---|
| Calc.(%) | C,57.79; | H,6.02; | N,6.97; | S,5.32 |
| Found(%) | C,57.98; | H,6.06; | N,7.02; | S,5.34 |

Example 2

Synthesis of 3-{4-diphenylmetylidyne-piperidin-1-yl}-propylcarbamoyl-3-oxothiomorpholine (Ib') and its oxalate (Ib)

The synthesis was conducted according to the following reaction scheme.

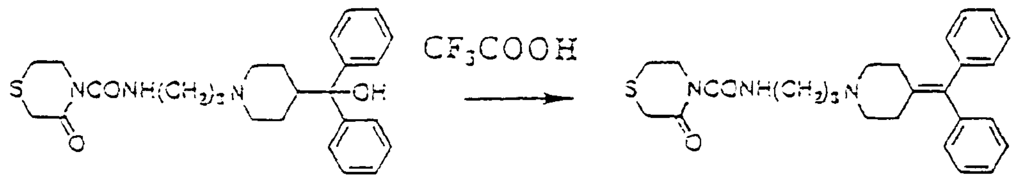


(Ia')  (Ib')

First, 15 ml of $CF_3COOH$ was added to 2 g (4.28 mmol) of Compound Ia' obtained in Example 1 and stirred at room temperature for 2 hours. $CF_3COOH$ was removed under reduced pressure, and $Na_2CO_3$ aqueous solution was added to the mixture so as to be alkaline. Then the mixture was extracted with dichloromethane. An organic layer of the extract was washed with water and saturated brine in this order, and dried, and the solvent was removed. The residue was purified by silica gel column chromatography using dichloromethaneisopropanol (15/1 v/v) to obtain 1.26 g of an oily product Ib' (yield: 87%).

IR($CHCl_3$)($cm^{-1}$) 3304, 3078, 2919, 2902, 2810, 1707, 1666, 1599, 1540

NMR($CDCl_3$)1.767(quint, j=7Hz), 2.30 to 2.60(m, 10H), 2.953(t, j=6Hz, 2H), 3.330(s, 2H), 3.372(q, j=6Hz, 2H), 4.189(t, j=6Hz, 2H), 7.06 to 7.35(m, 10H), 9.0 to 9.15(m, 1H)

Product Ib' was recrystallized from methanolisopropanol with conversion to an oxalate to obtain a purified product Ib as crystals having a melting point (d) of 189.0 to 190.0°C.

| Elem. Anal. for $C_{26}H_{31}N_3O_2S{\cdot}C_2H_2O_4$ FW 539.655 | | | | |
|---|---|---|---|---|
| Calc.(%) | C,62.32; | H,6.16; | N,7.79; | S,5.94 |
| Found(%) | C,62.41; | H,6.20; | N,7.83; | S,5.92 |

Example 3

Synthesis of 4-{3-(4-benzhydryl-piperazin-1-yl)-propylcarbamoyl}-3-oxothiomorpholine (Ic') and its maleate (Ic)

The synthesis was conducted according to the following reaction scheme.

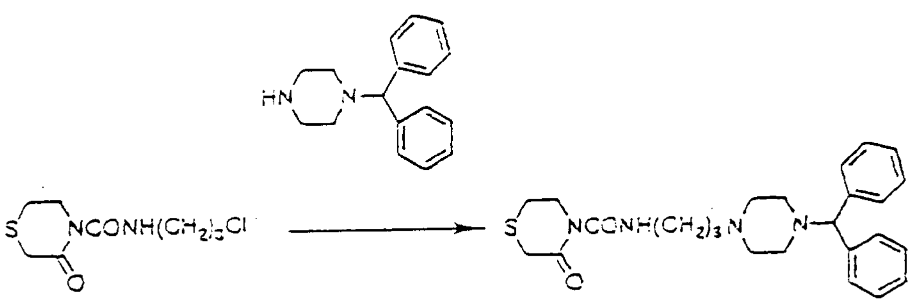

(Ic')

First, 2.5 g (10.6 mmol) of 4-(3-chloropropylcarbamoyl)3-oxothiomorpholine obtained in Reference Example 1 and 2.66 g (10.6 mmol) of 1-diphenylmethylpiperazine were dissolved in 25 ml of acetonitrile. To this mixture, 2.93 g (21.2 mmol) of $K_2CO_3$ and 0.88 g (5.3 mmol) of KI were added and heated with stirring at 90°C for 11.5 hours. Ethyl acetate was added to the reaction mixture, washed, and dried, and the solvent was removed. The residue was purified by silica gel column chromatography using dichloromethaneisopropanol (20/1 to 10/1 v/v) as an eluent to obtain 4.08 g of a crystalline product Ic' (yield: 85%). The product thus obtained was recrystallized with dichloromethane-ether to obtain crystals having a melting point of 79.60 to 81.5°C.

| Elem. Anal. for $C_{25}H_{32}N_4O_2S$ FW 452.622 | | | | |
|---|---|---|---|---|
| Calc.(%) | C,66.34; | H,7.13; | N,12.38; | S,7.08 |
| Found(%) | C,66.01; | H,7.16; | N,12.43; | S,6.94 |

IR($CHCl_3$)($cm^{-1}$) 3298, 3064, 2942, 2815, 1707, 1666, 1599, 1540,
NMR($CDCl_3$): 1.725(quint, j=5Hz, 2H), 2.30 to 2.65 (m, 10H), 2.962(t, j=6Hz, 2H), 3.337(s, 2H), 3.351(q, j=5Hz, 2H), 4.189(t, j=5Hz, 2H), 4.223(s, 1H), 7.10 to 7.50(m, 10H), 9.00 to 9.15(m, 1H)

Product Ic' was recrystallized from methanolisopropanol with conversion to a maleate to obtain a purified product Ic-1 as crystals having a melting point (d) of 176.5 to 177.5°C.

| Elem. Anal. for $C_{25}H_{32}N_4O_2S \cdot 2C_4H_4O_4$ FW 684.770 | | | | |
|---|---|---|---|---|
| Calc.(%) | C,57.88; | H,5.89; | N,8.18; | S,4.68 |
| Found(%) | C,57.79; | H,5.93; | N,8.14; | S,4.62 |

Product Ic' was recrystallized from methanolisopropanol-diethyl ether with conversion to a hydrochloride to obtain a purified product Ic-2 as crystals having a melting point (d) of 210.0 to 213.0°C.

| Elem. Anal. for $C_{25}H_{32}N_4O_2S \cdot 2HCl$ FW 525.544 | | | | | |
|---|---|---|---|---|---|
| Calc.(%) | C,57.14; | H,6.52; | N,10.66; | S,6.10; | Cl,13.49 |
| Found(%) | C,52.63; | H,5.66; | N,14.51; | S,13.23; | Cl,13.57 |

IR(Nujol)($cm^{-1}$) 3307(br), 2300(br), 1707, 1663, 1555, 1530, 1456

In each of the following Examples 4 to 84, Amine II which corresponds to the desired end product was allowed to react with Isocyanate III which also corresponds to the desired end product in accordance with the method described in Reference Example 1 to obtain Urea derivative IV. Then, Urea derivative IV was allowed to react with Amine V which corresponds to the desired end product in accordance with the method described in Example 1 and if required, the reaction product was dehydrated in accordance with the method described in Example 2 to obtain the following compounds.

Example 4

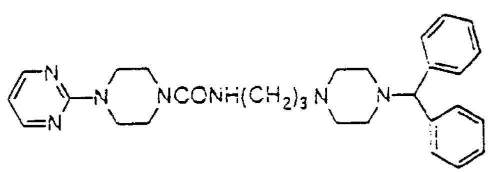

Recrystalized from methanol-isopropanol-diethyl ether
M.p. 117.0-120.0°C(d)

Elem. Anal. for $C_{29}H_{37}N_7O \cdot 2C_4H_4O_4$ FW 731.851

| | | | |
|---|---|---|---|
| Calc.(%) | C,60.73; | H,6.20; | N,13.40 |
| Found(%) | C,60.56; | H,6.20; | N,13.10 |

IR(Nujol)($cm^{-1}$) 3335,2380,2344,1698,1635,1583,1547

Example 5

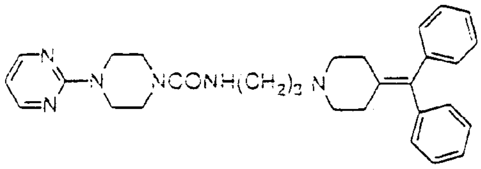

M.p. 159.0-160.5°C(d)

| Elem. Anal. for $C_{30}H_{36}N_6O \cdot C_2H_2O_4$ FW 586.696 | | | |
|---|---|---|---|
| Calc.(%) | C,65.51; | H,6.53; | N,14.32 |
| Found(%) | C,65.55; | H,6.53; | N,14.39 |

IR(Nujol)($cm^{-1}$) 1716,1655,1583,1544,1504,1451,1377, 1365,1244

Example 6

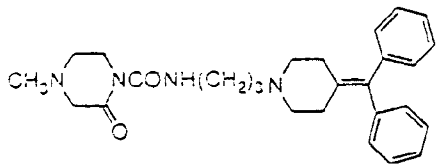

Recrystalized from methanol-isopropanol
M.p. 91.5-93.5°C(d)

| Elem. Anal. for $C_{27}H_{34}N_4O_2 \cdot 3/2C_2H_2O_4 \cdot 4/5H_2O$ FW 596.063 | | | |
|---|---|---|---|
| Calc.(%) | C,60.45; | H,6.53; | N,9.40 |
| Found(%) | C,60.46; | H,6.45; | N,9.42 |

IR(Nujol)($cm^{-1}$) 3488,3308,2683,2541(br),1709,1662,1623, 1523,1468,1377,1210

Example 7

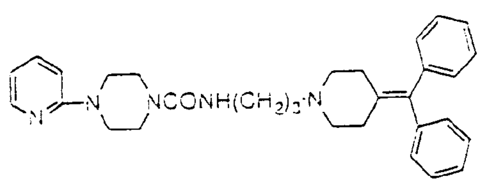

Recrystalized from ethyl acetate-diethyl ether
M.p. 158.0-159.5°C

| Elem. Anal. for $C_{31}H_{37}N_5O$ FW 495.672 | | | |
|---|---|---|---|
| Calc.(%) | C,75.12; | H,7.52; | N,14.13 |
| Found(%) | C,75.10; | H,7.56; | N,14.04 |

IR($CHCl_3$)($cm^{-1}$) 3274(br),3079,3056,3011,2893,1632,1597, 1564,1524,1482,1438,1240

Example 8

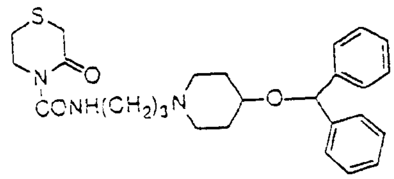

Recrystalized from methanol-isopropanol
M.p. 198.0-199.0°C(d)

| Elem. Anal. for $C_{26}H_{33}N_3O_3S{\cdot}C_2H_2O_4$ FW 557.670 | | | | |
|---|---|---|---|---|
| Calc.(%) | C,60.31; | H,6.33; | N,7.53; | S,5.75 |
| Found(%) | C,60.18; | H,6.34; | N,7.58; | S,5.68 |

IR(Nujol)($cm^{-1}$) 3308,2738,2664,2607,2526,1669, 1667,1608,1599,1524,1495,1455,1386,1205

Example 9

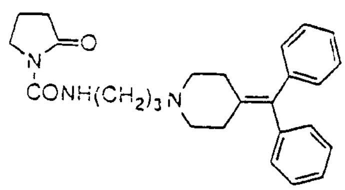

Recrystalized from methanol-isopropanol
M.p. 152.0-153.0°C(d)

| Elem. Anal. for $C_{26}H_{31}N_3O_2{\cdot}C_4H_4O_4$ FW 533.629 | | | |
|---|---|---|---|
| Calc.(%) | C,67.53; | H,6.61; | N,7.87 |
| Found(%) | C,67.50; | H,6.69; | N,7.85 |

IR(Nujol)($cm^{-1}$) 3314,2730,2603,2566,1707,1679,1618, 1528,1497

Example 10

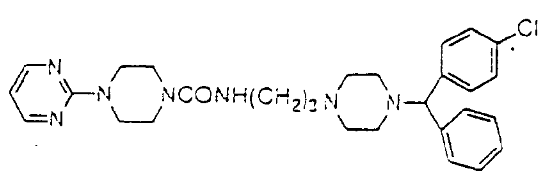

Recrystalized from ethyl acetate-isopropanol
M.p. 104.0-107.0°C(d)

| Elem. Anal. for $C_{29}H_{36}N_7OCl \cdot C_4H_4O_4 \cdot 1/10H_2O$ FW 768.056 | | | | |
|---|---|---|---|---|
| Calc.(%) | C,57.86; | H,5.80; | N,12.77; | Cl,4.62 |
| Found(%) | C,57.57; | H,5.87; | N,12.46; | Cl,4.69 |

IR(Nujol)($cm^{-1}$) 3320,2300(br),1695(br),1623,1583,1547, 1494,1451

Example 11

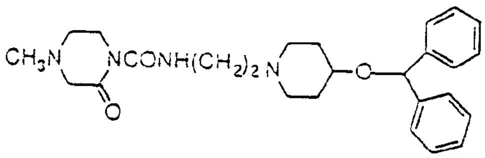

Recrystalized from methanol-isopropanol
M.p. 145.0-147.0°C(d)

| Elem. Anal. for $C_{26}H_{34}N_4O_3 \cdot 2C_2H_2O_4 \cdot 4/5H_2O$ FW 645.069 | | | |
|---|---|---|---|
| Calc.(%) | C,55.86; | H,6.19; | N,8.69 |
| Found(%) | C,55.99; | H,6.54; | N,8.48 |

IR(Nujol)($cm^{-1}$) 3318,2710(br),1703,1676,1636,1539,1496, 1456,1377,1219

Example 12

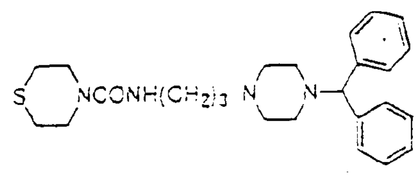

Recrystalized from methanol-isopropanol-diethyl ether
M.p. 218.0-228.0°C(d)

| Elem. Anal. for $C_{25}H_{34}N_4OS \cdot 2HCl \cdot 1/10H_2O$ FW 513.362 | | | | | |
|---|---|---|---|---|---|
| Calc.(%) | C,58.49; | H,7.11; | N,10.91; | S,6.25; | Cl,13.81 |
| Found(%) | C,58.72; | H,7.26; | N,10.59; | S,5.41; | Cl,13.37 |

IR(Nujol)($cm^{-1}$) 3348,2381(br),1633,1613,1531,1454,1427, 1399,1294,1251,1232

Example 13

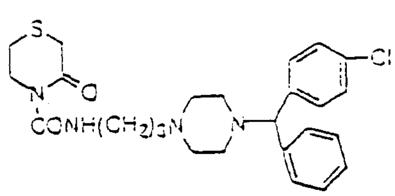

Recrystalized from diethyl ether-isopropanol
M.p. 160.0-161.0°C(d)

| Elem. Anal. for $C_{25}H_{31}N_4O_2SCl \cdot 2C_4H_4O_4$ FW 719.215 | | | | | |
|---|---|---|---|---|---|
| Calc.(%) | C,55.11; | H,5.47; | N,7.79; | S,4.46; | Cl,4.93 |
| Found(%) | C,55.05; | H,5.52; | N,7.93; | S,4.41; | Cl,4.98 |

IR(Nujol)($cm^{-1}$) 3268,2387(br),1705,1620,1533,1460,1385, 1346,1210

Example 14

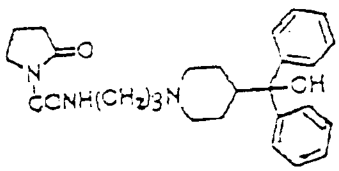

Recrystalized from diethyl ether-dichloromethane
M.p. 148.0-149.0°C

| Elem. Anal. for $C_{26}H_{33}N_3O_3 \cdot 0.4C_4H_{10}O$ FW 465.22 | | | |
|---|---|---|---|
| Calc.(%) | C,71.26; | H,8.02; | N,9.03 |
| Found(%) | C,70.95; | H,7.62; | N,9.55 |

Example 15

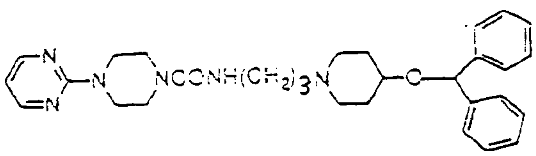

Recrystalized from diethyl ether-ethyl acetate
M.p. 124.5-125.5°C(d)

| Elem. Anal. for $C_{30}H_{38}N_6O_2$ FW 514.675 | | | |
|---|---|---|---|
| Calc.(%) | C,70.01; | H,7.44; | N,16.33 |
| Found(%) | C,69.90; | H,7.44; | N,16.07 |

IR($CHCl_3$)($cm^{-1}$) 3270,3030,2998,2948,2859,1634,1586, 1551,1496,1450

Example 16

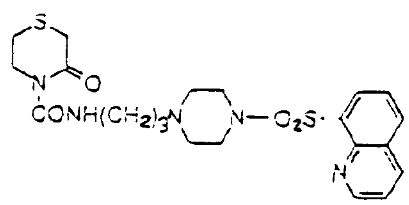

M.p. 129.0-130.5°C(d)

| Elem. Anal. for $C_{21}H_{27}N_5O_4S_2$ FW 477.608 | | | | |
|---|---|---|---|---|
| Calc.(%) | C,52.81; | H,5.70; | N,14.66; | S,13.43 |
| Found(%) | C,52.63; | H,5.66; | N,14.51; | S,13.23 |

IR($CHCl_3$)($cm^{-1}$) 3298,1707,1666,1612,1598,1541,1494, 1460,1387,1340,1162

Example 17

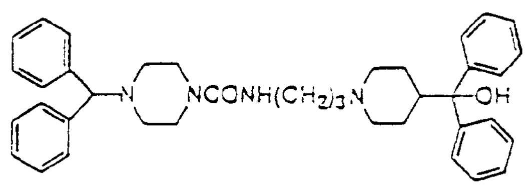

Recrystalized from methanol-isopropanol-diethyl ether
M.p. 195.5-200.0°C(d)

| Elem. Anal. for $C_{39}H_{46}N_4O_2 \cdot 2HCl \cdot 1.5H_2O$ FW 702.770 | | | | |
|---|---|---|---|---|
| Calc.(%) | C,66.66; | H,7.31; | N,7.97; | Cl,10.09 |
| Found(%) | C,66.69; | H,7.42; | N,7.97; | Cl,9.92 |

IR(Nujol)($cm^{-1}$) 3364(br),2567(br),1635,1544,1494,1455, 1377,1347,1285,1255

Example 18

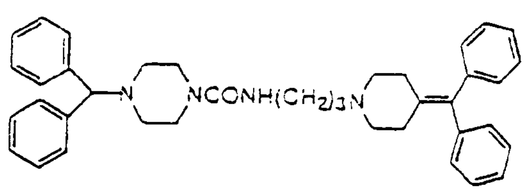

Recrystalized from isopropanol-diethyl ether
M.p.118.0-123.0°C(d)

| Elem. Anal. for $C_{39}H_{44}N_4O \cdot 2C_2H_2O_4 \cdot 1/2H_2O$ FW 773.890 | | | |
|---|---|---|---|
| Calc.(%) | C,66.74; | H,6.38; | N,7.24 |
| Found(%) | C,66.64; | H,6.48; | N,7.36 |

IR(Nujol) ($cm^{-1}$) 3334(br), 2598(br),1723, 1626, 1548, 1492, 1456, 1378, 1285, 1254, 1206

Example 19

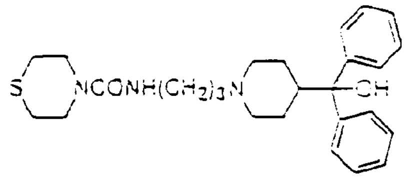

Recrystalized from isopropanol-diethyl ether
M.p. 110.0-135.0°C(d)

| Elem. Anal. for $C_{26}H_{35}N_3O_2S \cdot C_2H_2O_4 \cdot 1/2H_2O$ FW 552.694 | | | | |
|---|---|---|---|---|
| Calc.(%) | C,60.85; | H,6.93; | N,7.60; | S,5.80 |
| Found(%) | C,60.63; | H,6.84; | N,7.44; | S,4.94 |

IR(Nujol) ($cm^{-1}$) 3362(br),2534 (br),1735,1633,1534,1448, 1378,1254,1235

Example 20

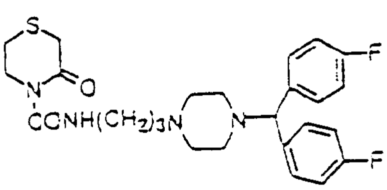

Recrystalized from methanol-isopropanol-diethyl ether
M.p. 215.0-216.0°C(d)

| Elem. Anal. for $C_{25}H_{30}N_4O_2SF_2 \cdot 2HCl \cdot 1/2H_2O$ FW 570.533 | | | | | | |
|---|---|---|---|---|---|---|
| Calc.(%) | C,52.63; | H,5.83; | N,9.82; | S,5.62; | Cl,12.43; | F,6.66 |
| Found(%) | C,52.89; | H,5.77; | N,9.78; | S,5.83; | Cl,12.56; | F,6.88 |

IR(Nujol)($cm^{-1}$) 3301,2817,1707,1666,1604,1540,1506, 1388,1232,1154

Example 21

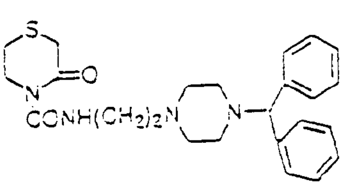

Recrystalized from methanol-isopropanol
M.p. 221.0-225.0°C(d)

| Elem. Anal. for $C_{24}H_{30}N_4O_2S \cdot 2HCl \cdot 1/4H_2O$ FW 516.021 | | | | | |
|---|---|---|---|---|---|
| Calc.(%) | C,55.86; | H,6.35; | N,10.86; | S,6.21; | Cl,13.74 |
| Found(%) | C,55.81; | H,6.73; | N,10.78; | S,6.15; | Cl,13.60 |

IR($CHCl_3$)($cm^{-1}$) 3295,2816,1706,1668,1599,1528,1388, 1227,1155

Example 22

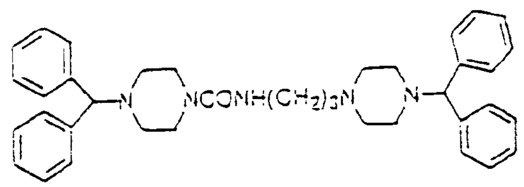

Recrystalized from methanol-isopropanol-diethyl ether
M.p. 115.0-125.0°C(d)

| Elem. Anal. for $C_{38}H_{45}N_5O \cdot 2C_2H_2O_4 \cdot H_2O$ FW 785.901 | | | |
|---|---|---|---|
| Calc.(%) | C,64.19; | H,6.54; | N,8.91 |
| Found(%) | C,64.03; | H,6.71; | N,8.74 |

IR(Nujol)($cm^{-1}$) 3317(br),2615(br),1723,1626,1549, 1455

Example 23

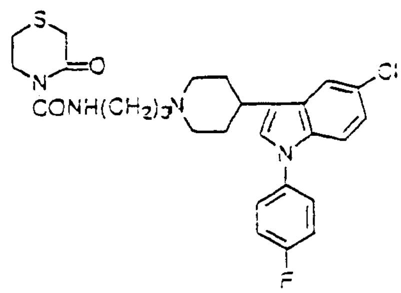

Recrystalized from methanol-isopropanol
M.p. 181.0-182.0°C(d)

| Elem. Anal. for $C_{27}H_{30}N_4O_2SFCl \cdot C_2H_2O_4 \cdot 1/2H_2O$ FW 628.124 | | | | | | |
|---|---|---|---|---|---|---|
| Calc.(%) | C,55.45; | H,5.30; | N,8.92; | S,5.10; | Cl,5.64; | F,3.02 |
| Found(%) | C,55.62; | H,5.42; | N,8.80; | S,5.11; | Cl,5.78; | F,3.36 |

IR(Nujol)($cm^{-1}$) 3273,2542,1714,1668,1625,1541,1513, 1459,1379,1205

Example 24

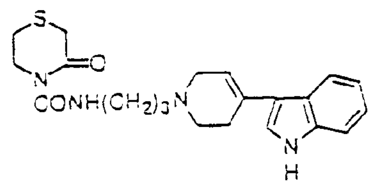

Recrystalized from methanol-isopropanol
M.p. 206.5-208.0°C(d)

| Elem. Anal. for $C_{21}H_{26}N_4O_2S \cdot C_2H_2O_4 \cdot 1/5H_2O$ FW 492.169 | | | | |
|---|---|---|---|---|
| Calc.(%) | C,56.13; | H,5.82; | N,11.38; | S,6.51 |
| Found(%) | C,56.12; | H,5.79; | N,11.36; | S,6.40 |

IR(Nujol)($cm^{-1}$) 3283,2538(br),1701,1667,1639,1616,1555, 1460,1389,1255,1212

Example 25

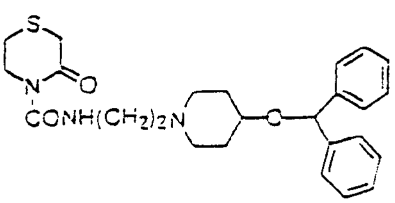

Recrystalized from methanol-isopropanol
M.p. 192.0-194.0°C(d)

Elem. Anal. for $C_{25}H_{31}N_3O_3S \cdot C_2H_2C_4$ FW 543.643

| | | | | |
|---|---|---|---|---|
| Calc.(%) | C,59.65; | H,6.12; | N,7.73; | S,5.90 |
| Found(%) | C,59.51; | H,6.15; | N,7.74; | S,5.81 |

IR(Nujol)($cm^{-1}$) 3316,2613(br),1721(sh),1670,1604,1533, 1456,1385,1207

Example 26

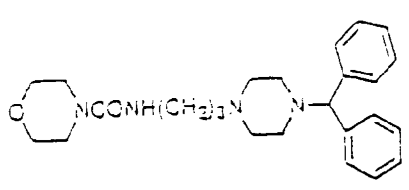

Recrystalized from methanol-isopropanol
M.p. 221.0-223.0°C(d)

| Elem. Anal. for $C_{25}H_{34}N_4O_2 \cdot 2HCl \cdot 1/4H_2O$ FW 500.000 | | | | |
|---|---|---|---|---|
| Calc.(%) | C,60.06; | H,7.36; | N,11.21; | Cl,14.18 |
| Found(%) | C,60.11; | H,7.39; | N,11.11; | Cl,14.09 |

IR($CHCl_3$)($cm^{-1}$) 3338,2381 (br),1631,1531,1454,1249,1107

Example 27

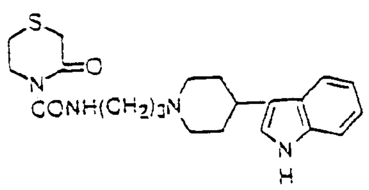

Recrystalized from methanol-isopropanol-diethylether
M.p. 203.0-204.0°C(d)

| Elem. Anal. for $C_{21}H_{28}N_4O_2S \cdot C_2H_2O_4 \cdot 1/4H_2O$ FW 495.086 | | | | |
|---|---|---|---|---|
| Calc.(%) | C,55.80; | H,6.21; | N,11.32; | S,6.48 |
| Found(%) | C,55.81; | H,6.24; | N,11.14; | S,6.19 |

IR(Nujol)($cm^{-1}$) 3316(br), 2519(br), 1700, 1640, 1617, 1556, 1460,1387,1208

Example 28

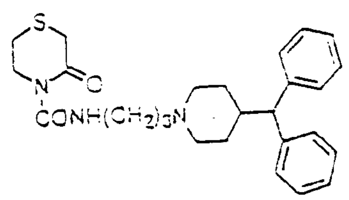

Recrystalized from methanol-isopropanol
M.p. 233.0-236.0°C(d)

| Elem. Anal. for $C_{26}H_{33}N_3O_2S \cdot HCl$ FW 488.096 | | | | | |
|---|---|---|---|---|---|
| Calc.(%) | C,63.98; | H,7.02; | N,8.61; | S,6.57; | Cl,7.26 |
| Found(%) | C,63.93; | H,7.14; | N,8.57; | S,6.45; | Cl,6.28 |

IR(Nujol)($cm^{-1}$) 3310,2481(br),1708,1660,1596,1529,1464, 1452,1386,1202,1122

Example 29

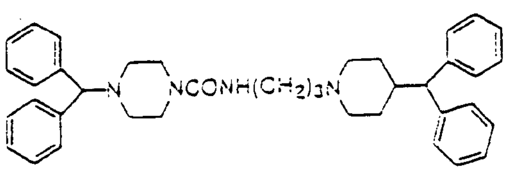

Recrystalized from methanol-isopropanol
M.p. 120.0-125.0°C(d)

| Elem. Anal. for $C_{39}H_{46}N_4O \cdot 2C_2H_2O_4$ FW 766.898 | | | |
|---|---|---|---|
| Calc.(%) | C,67.35; | H,6.57; | N,7.31 |
| Found(%) | C,67.05; | H,6.96; | N,7.15 |

IR(Nujol)(cm$^{-1}$) 3332(br),2616(br),1725,1625,1546,1454, 1377,1206

Example 30

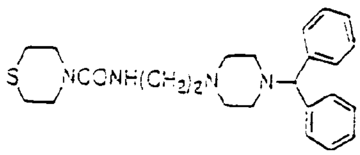

Recrystalized from methanol-isopropanol
M.p. 157.0-158.0°C(d)

| Elem. Anal. for $C_{24}H_{32}N_4OS \cdot C_2H_2O_4 \cdot 1/2H_2O$ FW 523.656 | | | | |
|---|---|---|---|---|
| Calc.(%) | C,59.64; | H,6.74; | N,10.70; | S,6.12 |
| Found(%) | C,60.00; | H,6.89; | N,10.65; | S,5.92 |

IR(Nujol)(cm$^{-1}$) 3375,1728(br),1632,1598,1558,1465,1452, 1377,1254

Example 31

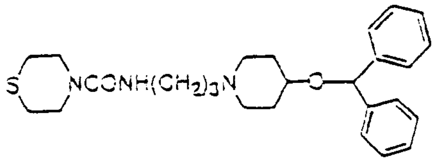

Recrystalized from methanol-isopropanol
M.p. 167.0-169.0°C(d)

| Elem. Anal. for $C_{26}H_{35}N_3O_2S \cdot C_2H_2O_4$ FW 543.686 | | | | |
|---|---|---|---|---|
| Calc.(%) | C,61.86; | H,6.86; | N,7.73; | S,5.90 |
| Found(%) | C,61.80; | H,7.02; | N,7.49; | S,5.39 |

IR(Nujol)(cm$^{-1}$) 3344(br),2736,2682(br),2621,2544,1718, 1621,1533,1454,1200

Example 32

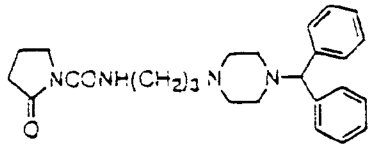

Recrystalized from methanol-isopropanol-diethyl ether

M.p. 153.0-157.0°C(d)

| Elem. Anal. for $C_{25}H_{32}N_4O_2$.2HCl.$H_2O$ FW 511.496 | | | | |
|---|---|---|---|---|
| Calc.(%) | C,58.71; | H,7.09; | N,10.95; | Cl,13.86 |
| Found(%) | C,58.71; | H,7.28; | N,10.93; | Cl,13.95 |

IR(Nujol)($cm^{-1}$) 3380(br),3323,2461(br),1703,1678,1626, 1523,1456,1381,1255

Example 33

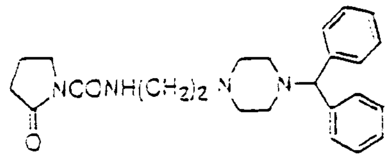

Recrystalized from methanol-isopropanol
M.p. 190.0-195.0°C(d)

| Elem. Anal. for $C_{24}H_{30}N_4O_2$·2HCl·$H_2O$ FW 497.469 | | | | |
|---|---|---|---|---|
| Calc.(%) | C,57.95; | H,6.89; | N,11.26; | Cl,14.25 |
| Found(%) | C,58.30; | H,7.06; | N,11.01; | S,14.19 |

IR(Nujol)(cm-1) 3300,2299(br),1713,1674,1548,1454,1433, 1384,1232

Example 34

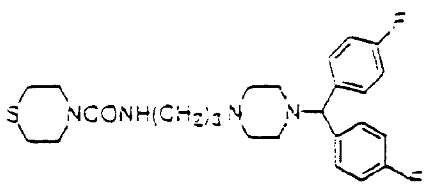

Recrystalized from methanol-isopropanol-diethyl ether
M.p. 153.5-154.5°C(d)

| Elem. Anal. for $C_{25}H_{32}N_4OSF_2$·$2C_4H_4O_4$ FW 706.768 | | | | | |
|---|---|---|---|---|---|
| Calc.(%) | C,56.08; | H,5.70; | N,7.93; | S,4.54; | F,5.38 |
| Found(%) | C,56.15; | H,5.70; | N,7.81; | S,4.53; | F,5.56 |

IR(Nujol)($cm^{-1}$) 3335,2390(br), 1694,1625,1606,1580, 1546, 1511,1450,1359,1237

Example 35

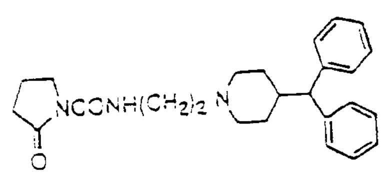

Recrystalized from methanol-isopropanol
M.p. 180.0-181.5°C(d)

| Elem. Anal. for $C_{25}H_{31}N_3O_2 \cdot C_2H_2O_4$ FW 495.580 | | | |
|---|---|---|---|
| Calc.(%) | C,65.44; | H,6.71; | N,8.48 |
| Found(%) | C,65.07; | H,6.84; | N,8.33 |

IR(Nujol)($cm^{-1}$) 3312,2670(br),1717,1646(br),1599,1523, 1463,1385,1268

Example 36

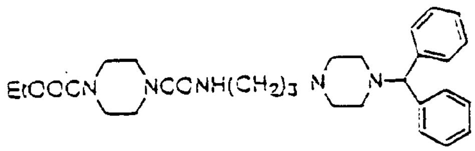

Recrystalized from isopropanol-diethyl ether
M.p. 107.0-117.0°C(d)

| Elem. Anal. for $C_{28}H_{39}N_5O_3 \cdot 2C_2H_2O_4 \cdot H_2O$ FW 691.740 | | | |
|---|---|---|---|
| Calc.(%) | C,55.56; | H,6.56; | N,10.12 |
| Found(%) | C,55.28; | H,6.38; | N,9.95 |

IR(Nujol)($cm^{-1}$) 3396,2550(br),1723(sh), 1699,1620,1531, 1459,1236

Example 37

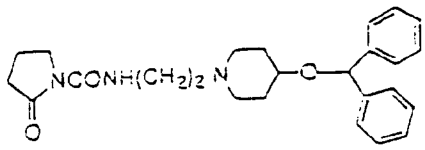

Recrystalized from methanol-isopropanol
M.p. 222.0-223.0°C(d)

| Elem. Anal. for $C_{25}H_{31}N_3O_3 \cdot C_2H_2O_4$ FW 511.579 | | | |
|---|---|---|---|
| Calc.(%) | C,63.39; | H,6.50; | N,8.21 |
| Found(%) | C,63.36; | H,6.58; | N,8.23 |

IR(Nujol)($cm^{-1}$) 3300,2599(br),1710,1680,1596,1540,1452, 1383

Example 38

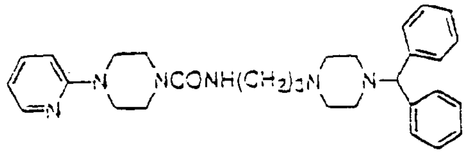

Recrystalized from methanol-diethyl ether

| Elem. Anal. for $C_{30}H_{38}N_6O \cdot 2C_2H_2O_4 \cdot 1.3H_2O$ FW 702.167 | | | |
|---|---|---|---|
| Calc.(%) | C,58.16; | H,6.40; | N,11.97 |
| Found(%) | C,58.26; | H,6.28; | N,11.73 |

IR(Nujol)($cm^{-1}$) 3355(br),2620(br),1722,1640,1612,1538, 1454,1378,1262,1238,1214,1179

Example 39

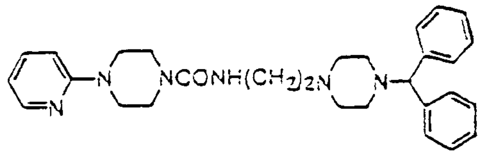

Recrystalized from methanol-diethyl ether

| Elem. Anal. for $C_{29}H_{36}N_6O \cdot 2C_2H_2O_4 \cdot 1.7H_2O$ FW 695.346 | | | |
|---|---|---|---|
| Calc.(%) | C,57.00; | H,6.29; | N,12.09 |
| Found(%) | C,57.08; | H,6.08; | N,11.56 |

IR(Nujol)($cm^{-1}$) 3357(br),2620(br),1722,1641,1623,1540, 1456,1379,1262,1238

Example 40

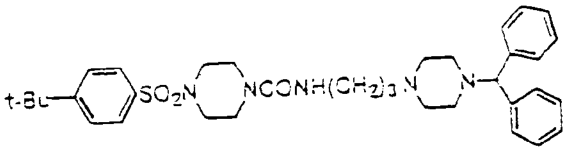

Recrystalized from methanol-isopropanol
M.p. 121.0-122.5°C(d)

| Elem. Anal. for $C_{35}H_{47}N_5O_3S \cdot 2C_4H_4O_4$ FW 850.007 | | | | |
|---|---|---|---|---|
| Calc.(%) | C,60.76; | H,6.52; | N,8.24; | S,3.77 |
| Found(%) | C,60.62; | H,6.63; | N,8.03; | S,3.60 |

IR(Nujol) ($cm^{-1}$) 3307,2347(br),1694,1631,1580,1549,1453, 1348,1262,1174

Example 41

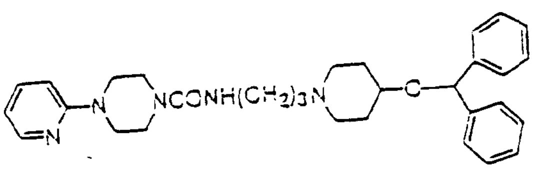

Recrystalized from methanol-isopropanol
M.p. 122.0-124.0°C(d)

| Elem. Anal. for $C_{31}H_{39}N_5O_2 \cdot C_2H_2O_4 \cdot 2H_2O$ FW 639.839 | | | |
|---|---|---|---|
| Calc.(%) | C,61.96; | H,7.09; | N,10.95 |
| Found(%) | C,61.90; | H,6.58; | N,10.54 |

IR(Nujol)(cm$^{-1}$) 3511(sh),3377,2737,2681,2625,2543,1724, 1643,1617,1546,1377,1208

Example 42

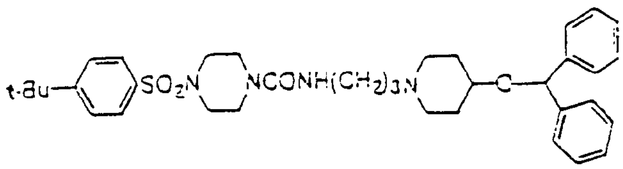

Recrystalized from acetone-diethyl ether
M.p. 122.0-125.0°C(d)

| Elem. Anal. for $C_{36}H_{48}N_4O_4S \cdot C_2H_2O_4 \cdot H_2O$ FW 740.922 | | | | |
|---|---|---|---|---|
| Calc.(%) | C,61.60; | H,7.07; | N,7.56; | S,4.33 |
| Found(%) | C,61.93; | H,6.92; | N,7.45; | S,4.01 |

IR(Nujol)(cm$^{-1}$) 3378(br),2734,2666,2620,2537,1718,1628, 1597,1540,1168

Example 43

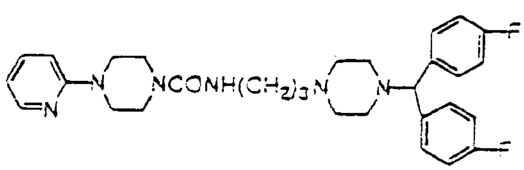

Recrystalized from isopropanol-diethyl ether

| Elem. Anal. for $C_{30}H_{36}N_6OF_2 \cdot 5/2C_4H_6O_6 \cdot 1/2H_2O$ FW 918.886 | | | | |
|---|---|---|---|---|
| Calc.(%) | C,52.29; | H,5.70; | N,9.15; | F,4.14 |
| Found(%) | C,52.15; | H,6.01; | N,8.79; | F,3.94 |

IR(Nujol ) (cm$^{-1}$) 3371(br),1726,1602(br),1543,1505,1461, 1379,1221

Example 44

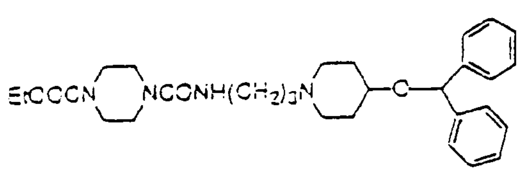

Recrystalized from isopropanol-diethyl ether
M.p. 121.0-123.0°C(d)

| Elem. Anal. for $C_{29}H_{40}N_4O_4{\cdot}C_2H_2O_4{\cdot}H_2O$ FW 616.716 | | | |
|---|---|---|---|
| Calc.(%) | C,60.38; | H,7.19; | N,9.08 |
| Found(%) | C,60.43; | H,7.45; | N,8.66 |

IR(Nujol)($cm^{-1}$) 3336,2719,2663,2626,2523,1693,1621, 1542,1460,1230

Example 45

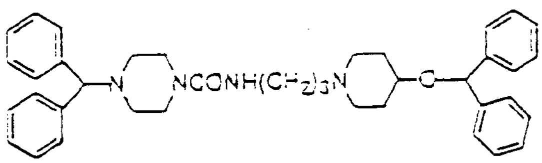

| Elem. Anal. for $C_{39}H_{46}N_4O_2{\cdot}2C_4H_6O_6{\cdot}3/2H_2O$ FW 930.026 | | | |
|---|---|---|---|
| Calc.(%) | C,60.70; | H,6.61; | N,6.02 |
| Found(%) | C,60.48; | H,6.69; | N,5.77 |

IR(Nujol)($cm^{-1}$) 3373,2587(br),1729,1601,1553,1455,1378, 1257,1124,1080

Example 46

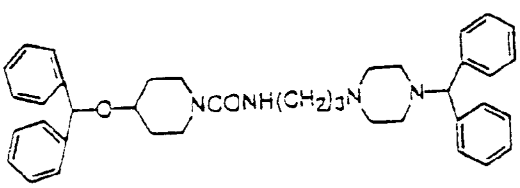

M.p. 141.0-143.0°C(d)

| Elem. Anal. for $C_{39}H_{46}N_4O_2{\cdot}2C_2H_2O_4{\cdot}3/4H_2O$ FW 796.409 | | | |
|---|---|---|---|
| Calc.(%) | C,64.85; | H,6.52; | N,7.03 |
| Found(%) | C,64.91; | H,6.49; | N,7.28 |

IR(Nujol)($cm^{-1}$) 3394,2553(br),1751,1722,1618,1538, 1457,1377,1280,1233

Example 47

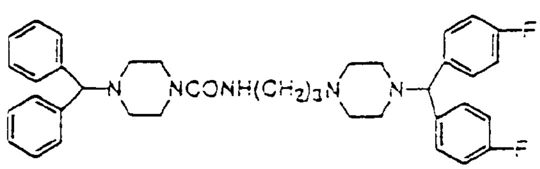

M.p. 128.0-130.0°C(d)

| Elem. Anal. for $C_{38}H_{43}N_5OF_2 \cdot 2C_2H_2O_4 \cdot 1/2H_20$ FW 812.874 | | | | |
|---|---|---|---|---|
| Calc.(%) | C,62.06; | H,5.95; | N,8.62; | F,4.67 |
| Found(%) | C,62.19; | H,6.10; | N,8.63; | F,4.88 |

IR(Nujol)($cm^{-1}$)
3293,2441 (br),1784,1709,1693,1647,1553,1505,1454,1228

Example 48

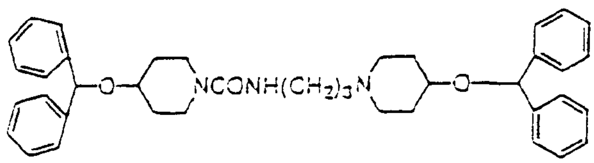

Recrystalized from methanol-isopropanol-diethyl ether
M.p. 151.0-153.0°C(d)

| Elem. Anal. for $C_{40}H_{47}N_3O_3 \cdot C_2H_2O_4$ FW 707.873 | | | |
|---|---|---|---|
| Calc.(%) | C,71.27; | H,6.98; | N,5.94 |
| Found(%) | C,71.27; | H,7.07; | N,5.96 |

IR(Nujol)($cm^{-1}$) 3400,2730,2658 (br),2550,1722,1695,1648, 1610,1527,1455,1200

Example 49

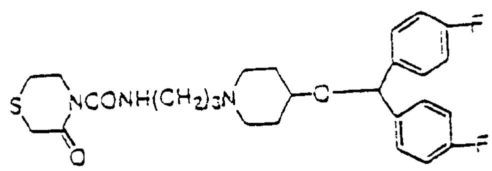

Recrystalized from methanol-isopropanol-diethyl ether
M.p. 176.0-177.0°C(d)

| Elem. Anal. for $C_{26}H_{31}N_3O_3SF_2 \cdot C_2H_2O_4$ FW 593.651 | | | | | |
|---|---|---|---|---|---|
| Calc.(%) | C,56.65; | H,5.60; | N,7.08; | S,5.40; | F,6.56 |
| Found(%) | C,56.53; | H,5.68; | N,6.89; | S,5.57; | F,6.56 |

IR(Nujol)($cm^{-1}$) 3292,2742,2667,2533,2449,1697,1663, 1606,1542,1508,1457,1382,1223,1204,1181

Example 50

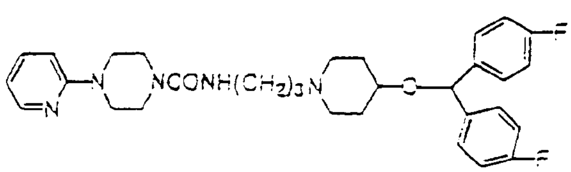

M.p. 113.0-118.0°C(d)

| Elem. Anal. for $C_{31}H_{37}N_5O_2F_2 \cdot 2C_2H_2O_4$ FW 729.740 | | | | |
|---|---|---|---|---|
| Calc.(%) | C,57.61; | H,5.66; | N,9.60; | F,5.21 |
| Found(%) | C,57.90; | H,6.27; | N,8.95; | F,4.93 |

IR(Nujol)($cm^{-1}$) 3357(br),2735,2663(br),2625,1716, 1625(br),1605,1541,1507,1459,1376,1226

Example 51

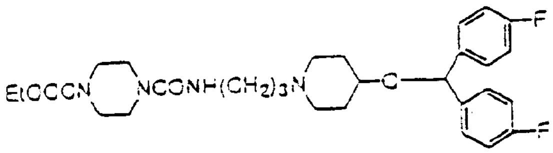

Recrystalized from isopropanol-diethyl ether
M.p. 151.0-152.0°C(d)

| Anal. for $C_{29}H_{38}N_4O_4F_2 \cdot C_2H_2O_4 \cdot 1/2Et_2O$ FW 671.744 | | | | |
|---|---|---|---|---|
| Calc.(%) | C,59.01; | H,6.75; | N,8.34; | F,5.66 |
| Found(%) | C,59.25; | H,6.36; | N,8.46; | F,6.45 |

IR(Nujol)($cm^{-1}$) 3360,2740,2670(br),2620,2540,1710, 1700,1625,1610,1540,1510,1460,1380,1230

Example 52

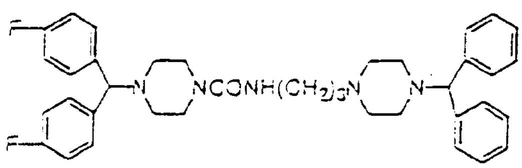

Recrystalized from methanol-isopropanol-diethyl ether
M.p. 114.0-116.0°C(d)

| Elem. Anal. for $C_{38}H_{43}N_5OF_2 \cdot 2C_2H_2O_4 \cdot 3/2H_2O$ FW 830.889 | | | | |
|---|---|---|---|---|
| Calc.(%) | C,60.71; | H,6.07; | N,8.43; | F,4.57 |
| Found(%) | C,60.96; | H,6.18; | N,8.27; | F,4.10 |

IR(Nujol)($cm^{-1}$) 3350(br),2610(br),1720,1700,1630,1610, 1550,1510,1450,1380,1280,1230

Example 53

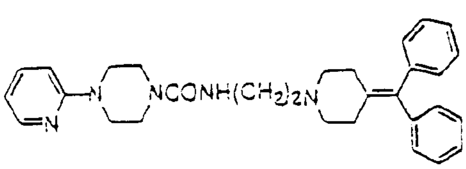

Recrystalized from dichloromethane-diethyl ether
M.p. 90.0-95.0°C

| Elem. Anal. for $C_{30}H_{35}N_5O\cdot3/5H_2O$ FW 492.454 | | | |
|---|---|---|---|
| Calc.(%) | C,73.17; | H,7.41; | N,14.22 |
| Found(%) | C,72.98; | H,7.43; | N,14.25 |

IR($CHCl_3$)($cm^{-1}$) 3410(br),1640,1600,1570,1510,1480,1440, 1240

Example 54

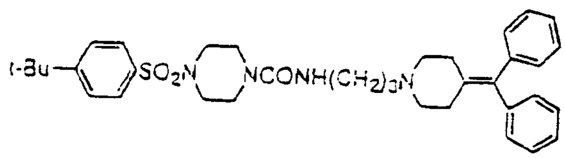

Recrystalized from methanol-acetone
M.p. 169.0-170.0°C(d)

| Elem. Anal. for $C_{36}H_{46}N_4O_3S\cdot C_2H_2O_4\cdot O.3H_2O$ FW 710.296 | | | | |
|---|---|---|---|---|
| Calc.(%) | C,64.26; | H,6.90; | N,7.89; | S,4.51 |
| Found(%) | C,64.00; | H,6.90; | N,7.74; | S,4.39 |

IR(Nujol)($cm^{-1}$) 3400,3330,2680,2600,2450,1780(sh),1715, 1640,1620,1595,1540,1460,1265,1175

Example 55

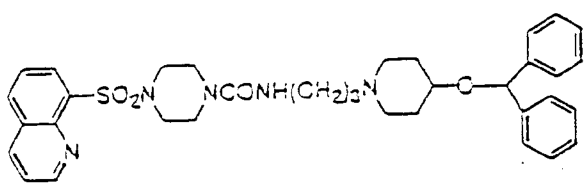

Recrystalized from methanol-isopropanol-diethyl ether
M.p. 125.0-127.5°C(d)

| Elem. Anal. for $C_{35}H_{41}N_5O_4S\cdot C_2H_2O_4\cdot1/2H_2O$ FW 726,854 | | | | |
|---|---|---|---|---|
| Calc.(%) | C,61.14; | H,6.10; | N,9.64; | S,4.41 |
| Found(%) | C,61.29; | H,6.34; | N,9.23; | S,4.05 |

IR(Nujol)($cm^{-1}$) 3350(br),2745,2680,2620,2550,1710,1700, 1620,1600,1540,1500,1450,1340,1265,1245

Example 56

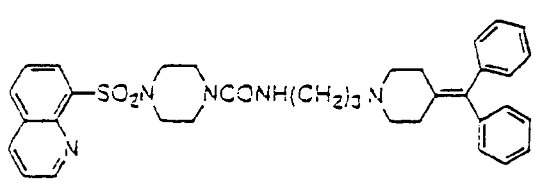

| Elem. Anal. for $C_{35}H_{39}N_5O_3S{\cdot}0.3C_6H_6$ FW 633.230 | | | | |
|---|---|---|---|---|
| Calc.(%) | C,69.80; | H,6.49; | N,11.06; | S,5.06 |
| Found(%) | C,69.65; | H,6.62; | N,10.81; | S,4.67 |

IR($CHCl_3$)($cm^{-1}$) 3280,1640,1600,1520,1495,1340,1320, 1160,1145

Example 57

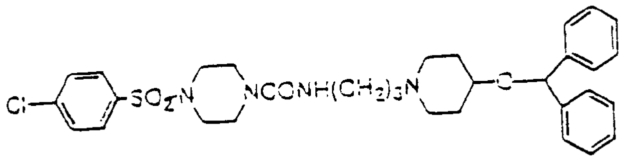

Recrystalized from methanol-isopropanol-diethyl ether
M.p. 119.0-121.0°C(d)

| Elem. Anal. for $C_{32}H_{39}N_4O_4SCl{\cdot}C_2H_2O_4{\cdot}1/2H_2O$ FW 710.251 | | | | | |
|---|---|---|---|---|---|
| Calc.(%) | C,57.50; | H,5.96; | N,7.89; | S,4.51; | Cl,4.99 |
| Found(%) | C,57.48; | H,5.88; | N,7.82; | S,4.42; | Cl,5.09 |

IR(Nujol)($cm^{-1}$) 3350,2740,2670,2630,2540,1710,1700, 1620,1590(sh),1540,1460,1355,1170

Example 58

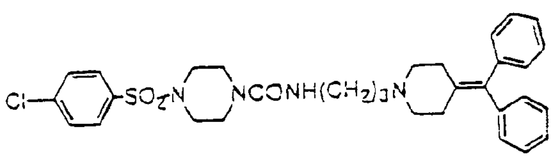

Recrystalized from dichloromethane-diethyl ether
M.p. 208.0-210.0°C

| Elem. Anal. for $C_{32}H_{37}N_4O_3SCl{\cdot}1/5H_2O$ FW 596.796 | | | | | |
|---|---|---|---|---|---|
| Calc.(%) | C,64.40; | H,6.32; | N,9.39; | S,5.37; | Cl,5.94 |
| Found(%) | C,64.34; | H,6.35; | N,9.39; | S,5.32; | Cl,6.25 |

IR($CHCl_3$)($cm^{-1}$) 3270(br),1640,1590,1530,1480,1360,1280, 1260,1170

Example 59

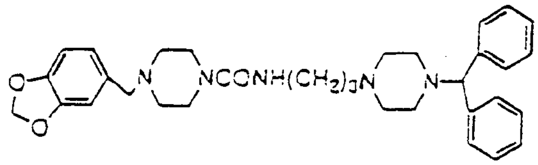

M.p. 107.0-110.0°C(d)

| Elem. Anal. for $C_{33}H_{41}N_5O_3 \cdot 2C_2H_2O_4 \cdot 3/2H_2O$ FW 762.820 | | | |
|---|---|---|---|
| Calc.(%) | C,58.26; | H,6.34; | N,9.18 |
| Found(%) | C,58.07; | H,6.35; | N,8.59 |

IR(Nujol)($cm^{-1}$) 3350(br),2600(br),1720,1700(sh), 1635, 1555,1450,1260,1210(br)

Example 60

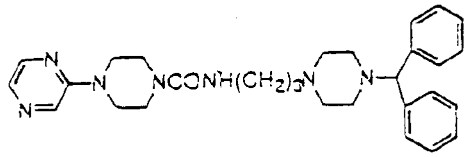

Recrystalized from methanol-isopropanol-diethyl ether
M.p. 164.0-166.0°C(d)

| Elem. Anal. for $C_{29}H_{37}N_7O \cdot 3C_2H_2O_4 \cdot 1/10H_2O$ FW 769.556 | | | |
|---|---|---|---|
| Calc.(%) | C,54.63; | H,5.40; | N,12.74 |
| Found(%) | C,54.33; | H,5.74; | N,12.49 |

IR(Nujol)($cm^{-1}$) 3380,2540(br),1720,1700(sh), 1640,1605, 1515,1460,1250,1240

Example 61

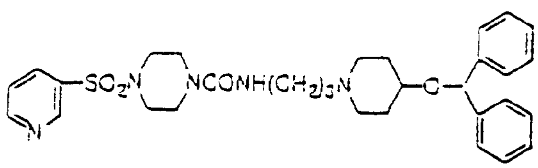

M.p. 114.0-116.0°C(d)

| Elem. Anal. for $C_{31}H_{39}N_5O_4S \cdot C_2H_2O_4 \cdot 2H_2O$ FW 703.817 | | | | |
|---|---|---|---|---|
| Calc.(%) | C,56.32; | H,6.44; | N,9.95; | S,4.56 |
| Found(%) | C,56.23; | H,6.15; | N,10.37; | S,4.98 |

IR(Nujol)($cm^{-1}$) 3350(br),2730,2670,2620,2540,1720, 1700,1630,1570,1540,1445,1420,1380,1350,1260

Example 62

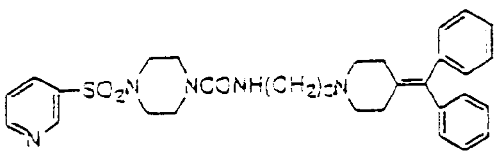

Recrystalized from dichloromethane-diethyl ether-n-hexane
M.p. 177.0-180.0°C

| Elem. Anal. for $C_{31}H_{37}N_5O_3S\cdot 1/10H_2O$ FW 561.537 | | | | |
|---|---|---|---|---|
| Calc.(%) | C,66.31; | H,6.68; | N,12.47; | S,5.71 |
| Found(%) | C,66.13; | H,6.75; | N,12.18; | S,5.61 |

IR($CHCl_3$)($cm^{-1}$) 3270(br),1640,1600,1575,1525,1420,1360, 1170

Example 63

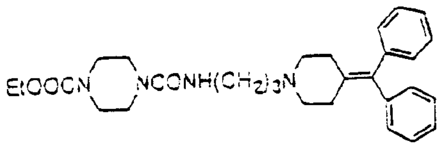

Recrystalized from dichloromethane-diethyl ether-n-hexane
M.p. 128.0-133.0°C

| Elem. Anal. for $C_{29}H_{38}N_4O_3\cdot 1/5H_2O$ FW 494.253 | | | |
|---|---|---|---|
| Calc.(%) | C,70.47; | H,7.83; | N,11.34 |
| Found(%) | C,70.51; | H,7.81; | N,11.20 |

IR($CHCl_3$) ($cm^{-1}$) 3270(br),1685,1630,1520,1435,1250,1240

Example 64

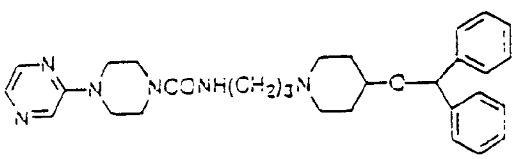

Recrystalized from methanol-isopropanol-diethyl ether
M.p. 159.0-160.0°C(d)

| Elem. Anal. for $C_{30}H_{38}N_6O_2.C_2H_2O_4\cdot 1/2H_2O$ FW 613.719 | | | |
|---|---|---|---|
| Calc.(%) | C,62.63; | H,6.73; | N,13.69 |
| Found(%) | C,62.86; | H,6.68; | N,13.38 |

IR(Nujol)($cm^{-1}$) 3410,3350(br),2745,2670(br),2620,2540, 1720,1695,1640,1620,1600,1580,1520,1460,1250

Example 65

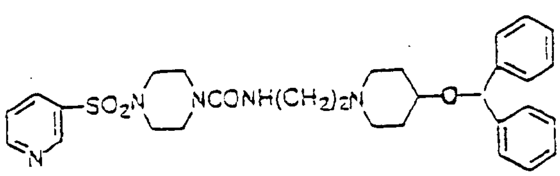

Recrystalized from methanol-isopropanol-diethyl ether
M.p. 170.0-172.5°C(d)

| Elem. Anal. for $C_{30}H_{37}N_5O_4S \cdot C_2H_2O_4 \cdot 5/4H_2O$ FW 676.278 | | | | |
|---|---|---|---|---|
| Calc.(%) | C,56.83; | H,6.19; | N,10.36; | S,4.74 |
| Found(%) | C,56.95; | H,6.02; | N,10.87 | |

IR(Nujol)($cm^{-1}$) 3350(br),2750,2690,1720,1700(sh),1655, 1535,1455,1410,1350,1260,1175

Example 66

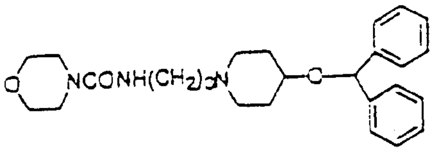

Recrystalized from methanol-isopropanol-diethyl ether
M.p. 157.5-158.5°C(d)

| Elem. Anal. for $C_{26}H_{35}N_3O_3 \cdot C_2H_2O_4$ FW 527.622 | | | |
|---|---|---|---|
| Calc.(%) | C,63.74; | H,7.07; | N,7.96 |
| Found(%) | C,63.71; | H,7.14; | N,7.71 |

IR(Nujol)($cm^{-1}$) 3400,3350(sh),2750,2690,2620,2550,1720, 1695,1650,1610,1515,1460,1195

Example 67

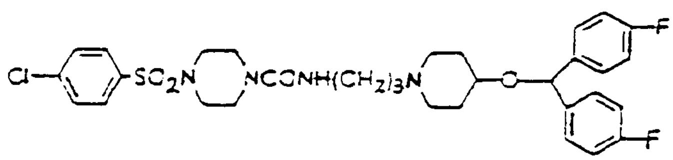

M.p. 135.0-140.0°C(d)

| Elem. Anal. for $C_{32}H_{37}N_4O_4SClF_2 \cdot C_2H_2O_4 \cdot 1/2H_2O$ FW 746.232 | | | | | | |
|---|---|---|---|---|---|---|
| Calc.(%) | C,54.73; | H,5.40; | N,7.51; | S,4.30; | Cl,4.75; | F,5.09 |
| Found(%) | C,54.94; | H,5.29; | N,7.47; | S,4.70; | Cl,5.10; | F,5.13 |

IR(Nujol)($cm^{-1}$) 3375(br),2740,2680,2620,2550,1720,1700, 1630,1605,1540,1510,1460,1220,1170

Example 68

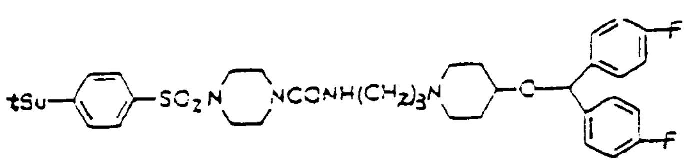

Recrystalized from methanol-isopropanol-diethyl ether
M.p. 145.0-147.5°C(d)

| Elem. Anal. for $C_{36}H_{46}N_4O_4SF_2 \cdot C_2H_2O_4 \cdot 1/4H_2O$ FW 763.391 | | | | | |
|---|---|---|---|---|---|
| Calc.(%) | C,59.79; | H,6.40; | N,7.34; | S,4.20; | F,4.98 |
| Found(%) | C,59.60; | H,6.34; | N,7.30; | S,4.36; | F,4.96 |

IR(Nujol) ($cm^{-1}$) 3370(br),2740,2680,2630,2550,1720,1700, 1630,1610,1540,1510,1460,1350,1220,1170

Example 69

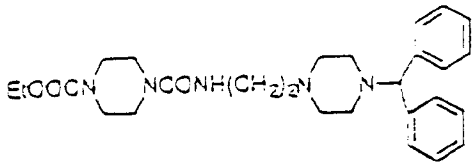

Recrystalized from methanol-isopropanol-diethyl ether
M.p. 130.5-134.0°C(d)

| Elem. Anal. for $C_{27}H_{37}N_5O_3 \cdot C_2H_2O_4$ FW 571.678 | | | |
|---|---|---|---|
| Calc.(%) | C,60.93; | H,7.23; | N,12.25 |
| Found(%) | C,59.06; | H,7.02; | N,11.08 |

IR( Nujol)($cm^{-1}$) 3370(br),2670,2530,1760(sh),1700,1620, 1600,1560,1455,1380,1250,1240

Example 70

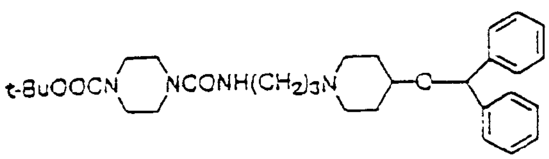

Recrystalized from methanol-isopropanol-diethyl ether
M.p. 150.5-151.5°C(d)

| Elem. Anal. for $C_{31}H_{44}N_4O_4 \cdot C_2H_2O_4$ FW 626.755 | | | |
|---|---|---|---|
| Calc.(%) | C,63.24; | H,7.40; | N,8.94 |
| Found(%) | C,63.21; | H,7.35; | N,8.80 |

IR(Nujol)($cm^{-1}$) 3400,3380,2740,2680,2620,2530,1720, 1695,1670,1640,1600(sh),1545,1455,1415,1240

Example 71

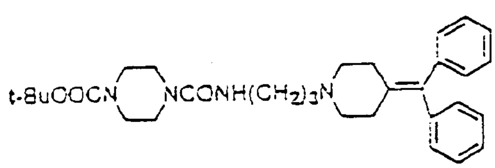

Recrystalized from dichloromethane-diethyl ether
M.p. 148.5-150.0°C

| Elem. Anal. for $C_{31}H_{42}N_4O_3 \cdot C_2H_2O_4$ FW 518.70 | | | |
|---|---|---|---|
| Calc.(%) | C,71.78; | H,8.16; | N,10.80 |
| Found(%) | C,71.44; | H,8.17; | N,10.73 |

IR($CHCl_3$)($cm^{-1}$) 3275(br),1685,1630,1600(sh),1525,1420, 1250,1240,1170

Example 72

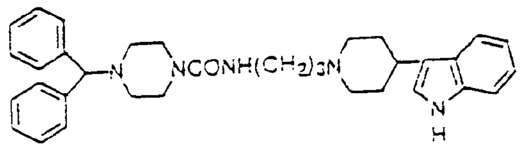

| Elem. Anal. for $C_{34}H_{41}N_5O \cdot 2C_2H_2O_4$ FW 715.809 | | | |
|---|---|---|---|
| Calc.(%) | C,63.76; | H,6.34; | N,9.78 |
| Found(%) | C,63.47; | H,6.61; | N,9.99 |

IR(Nujol)($cm^{-1}$) 3300(br),2570(br),1720,1700(sh),1630, 1550,1455,1380,1250

Example 73

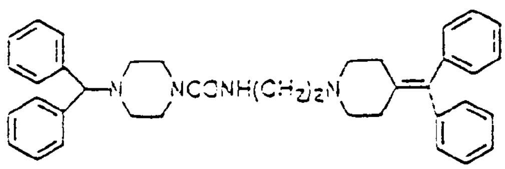

| Elem. Anal. for $C_{38}H_{42}N_4O \cdot 2C_2H_2O_4 \cdot 1/2H_2O$ FW 759.863 | | | |
|---|---|---|---|
| Calc.(%) | C,66.39; | H,6.23; | N,7.37 |
| Found(%) | C,66.10; | H,6.52; | N,7.32 |

IR(Nujol)($cm^{-1}$) 3330(br),2600(br),1720,1630,1540,1495, 1455,1380,1285,1255,1205

Example 74

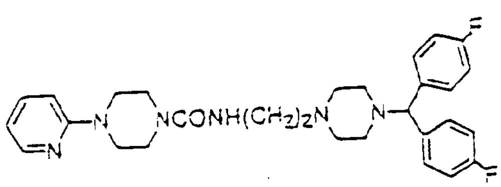

Recrystalized from methanol-isopropanol
M.p. 197.0-198.0°C(d)

| Elem. Anal. for $C_{29}H_{34}N_6OF_2 \cdot C_4H_4O_4 \cdot H_2O$ FW 654.719 | | | | |
|---|---|---|---|---|
| Calc.(%) | C,60.54; | H,6.16; | N,12.84; | F,5.80 |
| Found(%) | C,60.53; | H,5.94; | N,12.04; | F,5.47 |

IR(Nujol)($cm^{-1}$) 3270,2600,2520(br),1710,1690,1640, 1580,1540,1500,1475,1435,1275,1220,1200

Example 75

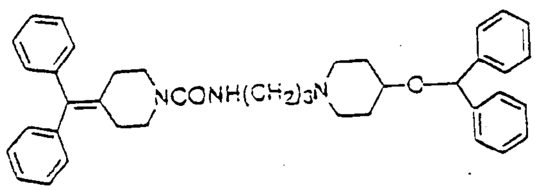

Recrystalized from methanol-isopropanol
M.p. 124.0-126.0°C(d)

| Elem. Anal. for $C_{40}H_{45}N_3O_2 \cdot C_2H_2O_4 \cdot H_2O$ FW 707.873 | | | |
|---|---|---|---|
| Calc.(%) | C,71.27; | H,6.98; | N,5.94 |
| Found(%) | C,71.17; | H,6.77; | N,5.83 |

IR(Nujol)($cm^{-1}$) 3350(br),2730,2680,2620,2545,1720,1700, 1625,1600(sh),1540,1450,1375,1235,1190

Example 76

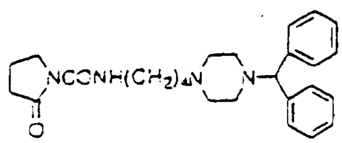

Recrystalized from methanol-isopropanol-diethyl ether
M.p. 153.0-154.0°C

| Elem. Anal. for $C_{26}H_{34}N_4O_2 \cdot C_2H_2O_4 \cdot$ 1/2i-PrOH$\cdot$1/3$H_2O$ FW 560.671 | | | |
|---|---|---|---|
| Calc.(%) | C,63.26; | H,7.31; | N,10.00 |
| Found(%) | C,63.29; | H,7.44; | N,9.82 |

IR(Nujol)($cm^{-1}$) 3472,3285,2606,2548,1712,1679,1639, 1554,1489,1452

Example 77

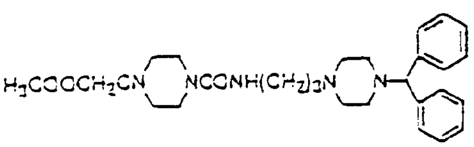

Recrystalized from methanol-isopropanol-diethyl ether
M.p. 112.0-114.0°C

| Elem. Anal. for $C_{28}H_{39}N_5O_3 \cdot 2C_2H_2O_4 \cdot 1.4H_2O$ FW 698.947 | | | |
|---|---|---|---|
| Calc.(%) | C,54.99; | H,6.60; | N,10.02 |
| Found(%) | C,55.02; | H,6.40; | N,9.92 |

IR(KBr)($cm^{-1}$) 3412,2933,2567,1738,1634,1545,1497,1455

Example 78

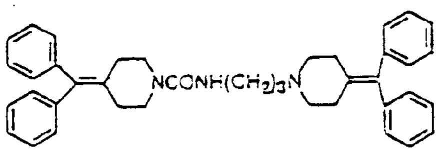

Recrystalized from methanol-isopropanol-diethyl ether
M.p. 150.0-160.0°C(d)

| Elem. Anal. for $C_{40}H_{43}N_3O \cdot C_4H_6O_6 \cdot 0.5H_2O$ FW 740.903 | | | |
|---|---|---|---|
| Calc.(%) | C,71.33; | H,6.80; | N,5.67 |
| Found(%) | C,71.41; | H,7.10; | N,5.46 |

IR(Nujol)($cm^{-1}$) 3440,3330,1745,1715,1600,1545,1450

Example 79

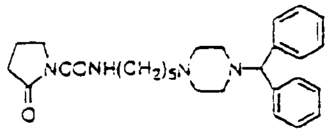

Recrystalized from methanol-isopropanol-diethyl ether
M.p. 160.0-161.0°C(d)

| Elem. Anal. for $C_{27}H_{36}N_4O_2 \cdot C_2H_2O_4 \cdot 1/3H_2O$ FW 544.647 | | | |
|---|---|---|---|
| Calc.(%) | C,63.95; | H,7.16; | N,10.29 |
| Found(%) | C,63.93; | H,7.40; | N,9.89 |

IR(Nujol)($cm^{-1}$) 3300,1710,1680,1550,1455,1375

Example 80

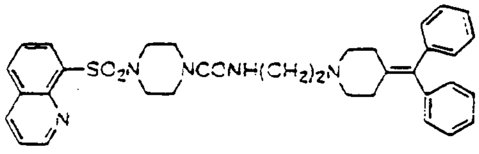

Recrystalized from isopropanol-diethyl ether
M.p. 147.0-148.5°C

| Elem. Anal. for $C_{34}H_{37}N_5O_3S \cdot 1/10H_2O$ FW 597.570 | | | |
|---|---|---|---|
| Calc.(%) | C,68.34; | H,6.27; | N,11.72 |
| Found(%) | C,68.15; | H,6.38; | N,11.68 |

IR(Nujol)($cm^{-1}$) 3411,1638,1598,1508,1341,1261,1162,1144

Example 81

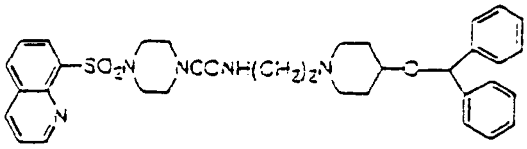

| Elem. Anal. for $C_{34}H_{39}N_5O_4S \cdot C_2H_2O_4 \cdot 5/4H_2O$ FW 726.339 | | | | |
|---|---|---|---|---|
| Calc.(%) | C,59.53; | H,6.04; | N,9.64; | S,4.41 |
| Found(%) | C,59.35; | H,5.90; | N,10.14; | S,4.60 |

IR(Nujol)($cm^{-1}$) 3395,1719,1644,1544,1455,1339,1264, 1162,1145

Example 82

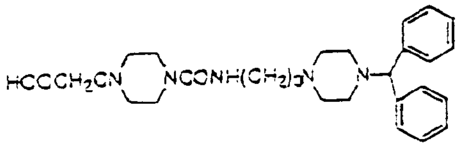

| Elem. Anal.for $C_{27}H_{37}N_5O_3 \cdot 3HCl \cdot 1/2i\text{-}PrOH \cdot 7/10H_2O$ FW 631.667 | | | | |
|---|---|---|---|---|
| Calc.(%) | C,54.19; | H,7.24; | N,11.09; | Cl,16.84 |
| Found(%) | C,53.92; | H,7.13; | N,11.38; | Cl,16.54 |

IR(KBr) ($cm^{-1}$) 3421,2933,2567,1738,1634,1545,1497,1455

Example 83

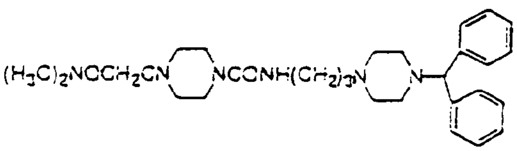

Recrystalized from ethyl acetate
M.p.136.5-139.0°C

| Elem. Anal. for $C_{29}H_{42}N_6O_2 \cdot 7/10H_2O$ FW 519.306 | | | |
|---|---|---|---|
| Calc.(%) | C,67.07; | H,8.42; | N,16.18 |
| Found(%) | C,67.05; | H,8.29; | N,16.27 |

IR($CHCl_3$) ($cm^{-1}$) 3286,1636,1523,1493,1451,1403

Example 84

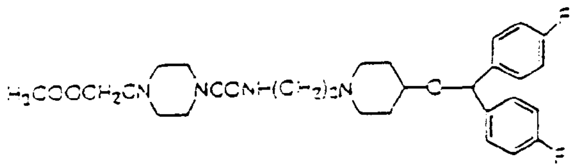

Recrystalized from methanol
M.p. 166.5-167.5°C(d)

| Elem. Anal. for $C_{29}H_{38}N_4F_2O_4 \cdot 2C_2H_2O_4 \cdot 2/5H_2O$ FW 731.924 | | | | |
|---|---|---|---|---|
| Calc.(%) | C,54.15; | H,5.89; | N,7.65; | F,5.19 |
| Found(%) | C,54.10; | H,5.82; | N,7.59; | F,5.13 |

IR(Nujol)($cm^{-1}$) 3433,2741,2669,2627,2549,1749,1710, 1693,1648,1605,1460,1440,1402

Example 85

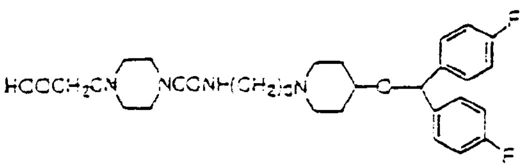

Recrystalized from methanol-isopropanol
M.p. 148.0-150.0°C(d)

| Elem. Anal. for $C_{28}H_{36}N_4F_2O_4 \cdot 3/2C_2H_2O_4 \cdot H_2O$ FW 683.688 | | | | |
|---|---|---|---|---|
| Calc.(%) | C,54.46; | H,6.04; | N,8.19; | F,5.56 |
| Found(%) | C,54.55; | H,5.98; | N,8.34; | F,5.40 |

IR(Nujol)($cm^{-1}$) 3415,2738,2666,2625,2546,1908,1735, 1716,1695,1606,1546, 1508,1459,1405

Example 86

Evaluation of antihistaminic effects in vitro

The following experiments were conducted in accordance with the method of R. Magnus (Arch. F.D. Ges. Physiol., 102: 123-151, 1904). Ileums derived from guinea pigs were respectively soaked in a saline solution at 37°C. Then, histamine diphosphate and a subject compound which had been obtained in the Examples shown in Table 1 were added to each saline solution so that the concentrations of histamine diphosphate and the subject compound are 0.5 μg/ml and 0.03 to 10 μg/ml, respectively. The ileum generally contracts by histamine; however, in the case where the subject compound is present, the antagonistic effect of the subject compound on histamine inhibits the contraction of the ileum. The contraction degree of the ileum was measured at each concentration of the added subject compound, and the concentration $IC_{50}$ of the subject compound which inhibited 50% submaximal contraction of the ileum by histamine was obtained. The submaximal contraction was obtained in the case where the subject compound was not added. The obtained concentration $IC_{50}$ was shown in Table 1. Table 1 also shows the measurements regarding the known antihistaminic agents, terphenadine and oxatomide. When the value of $IC_{50}$ is lower, the subject cmpound has stronger antihistaminic effects.

Example 87

$H_1$ receptor binding inhibition test in vitro

In order to evaluate the affinity of the compound of the present invention for a $H_1$ receptor, the following experiment was conducted.

Three month old Slc-Wistar rats were killed by decapitation, and its cerebral cortex was rapidly removed. The cerebral cortex was homogenized with about 20 times its volume of 50 mM of ice-cooled Na/K phosphate buffer (pH 7.5). The homogenate was centrifuged at 40000 g for 10 minutes, and then, resuspended in the buffer. This process was repeated three times, and the resulting suspension was kept in a refrigerator at -80°C. This sample was thawed at 4°C before experiment. Then, the homogenate was centrifuged and resuspended to obtain a receptor sample. The compound of the present invention listed in Table 1 was added in the range of 0.1 nM to 100 μM to a mixture of the receptor sample and [$^3$H]-pyrilamine (4 nM) and incubated at 25°C for 30 minutes. The reaction was stopped by dilution and filtration. The radioactivity of [$^3$H]-pyrilamine bound to the receptor sample on the filter paper was measured by a liquid scintillation counter. The specific binding of [$^3$H]-pyrilamine to the receptor sample was obtained by subtracting the non-specific binding from the total bindings. Using the concentration $IC_{50}$ of the compound of the present invention inhibiting 50% specific binding of [$^3$H]-pyrilamine to the receptor sample, an inhibition constant Ki was obtained from the following equation.

$$Ki = \frac{IC_{50}}{1 + C/Kd}$$

where C is the concentration of [$^3$H]- pyrilamine; and Kd is the dissociation constant.

The results are shown in Table 1. The smaller the value that Ki is, the stronger affinity the compound has for the histamine receptor.

Example 88

$H_1$ receptor binding inhibition test ex vivo

In order to evaluate the migration of the compound of the present invention to the brain, the following experiment was conducted.

Five Slc-ddY male mice were used as one group. First, 10 mg/kg of the compound of the present invention was orally administered to each mouse. The mouse were killed by decapitation 60 minutes after the administration and each cerebral cortex was removed. Each cerebral cortex was frozen on dry ice and kept in a refrigerator at -80°C. Before the experiment, each cerebral cortex was homogenized with about 10 times its volume of 50 mM of Na/K phosphate buffer (pH 7.5). A mixture of each cerebral cortex was further diluted 6 times and used as a receptor sample. A mixture of each receptor sample and [$^3$H]-pyrilamine (4 nM) was incubated at 25°C for 30 minutes. The reaction was stopped by dilution and filtration. The radioactivity of [$^3$H]-pyrilamine bound to the receptor sample on the filter paper was measured by a liquid scintillation counter. The specific binding of [$^3$H]-pyrilamine to the receptor sample was obtained by subtracting the non-specific binding from the total bindings. The non-specific binding was obtained under the conditions of containing 10 μM of non-radioactive pyrilamine. The amount of protein was measured by DC protein assay (Biolad), and the inhibition of the compound of the present invention to the specific binding of [$^3$H]-pyrilamine per 1 mg of protein was measured. The results are shown in Table 1. The lower the inhibition is, the lower migration to a brain the compound has.

Table 1

| Compound | Antihistaminic effect $IC_{5\emptyset}$(μg/ml) | Affinity for $H_1$ receptor in vitro Ki (μM) | [$^3$H]-labeled pyrilamine binding inhibition ex vivo (%) |
|---|---|---|---|
| Terfenadine | 22.367 | 0.33 | 9 |
| Oxatoamide | 1 | 0.066 | 17 |
| Example 5 | 0.442 | 0.094 | 0 |
| Example 15 | 0.0595 | 0.043 | 6 |
| Example 39 | 0.119 | 0.06 | 4 |
| Example 43 | 0.216 | 0.057 | 14 |
| Example 45 | 0.621 | 0.22 | 6 |
| Example 49 | 0.0244 | 0.027 | 9 |
| Example 50 | 0.0817 | 0.042 | 2 |
| Example 51 | 0.427 | 0.038 | 0 |
| Example 53 | 1.764 | 0.12 | 0 |
| Example 55 | 1.142 | 0.018 | 0 |
| Example 56 | 1.376 | 0.14 | 0 |
| Example 61 | 0.196 | 0.049 | 6 |
| Example 70 | 0.0889 | 0.032 | 2 |
| Example 71 | 1.122 | 0.15 | 7 |
| Example 79 | 0.042 | 0.042 | 5 |

From Table 1, the compound of the present invention was confirmed to have high affinity for the histamine receptor and low migration to the brain. Thus, it is considered that the compound of the present invention involves less side-effects while having strong antihistaminic effects.

The compound of the present invention has a repressing effect such as an antagonistic effect on various chemical mediators in addition to histamine.

As described above, according to the present invention, a novel compound useful as an antiallergic agent is provided. The compound has a strong antihis-taminic effect and involvies less side effects. The compound of the present invention can also be used in various fields because of its repressing effect such as an antagonistic effect on various chemical mediators.

Various other modifications will be apparent to and can be readily made by those skilled in the art without departing from the scope and spirit of this invention. Accordingly, it is not intended that the scope of the claims appended hereto be limited to the description as set forth herein, but rather that the claims be broadly construed.

## Claims

1. A compound represented by the following Formula I :

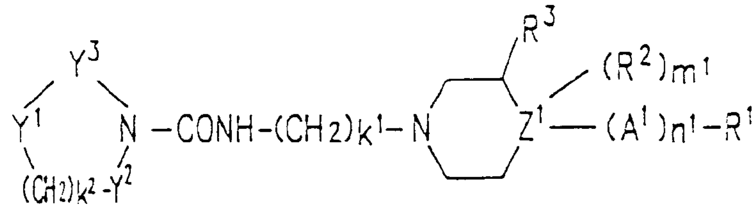

(I)

wherein $Z^1$ is C or N; $m^1$ is 0 when $Z^1$ is N and $m^1$ is 1 when $Z^1$ is C; $R^3$ can form a bond with $R^2$ when $Z^1$ is C; $A^1$ is O, $SO_2$, or $CH_2$; $n^1$ is 0 or 1 when $A^1$ is O or $SO_2$ and $n^1$ is an integer of 0 to 3 when $A^1$ is $CH_2$; $R^1$ is a group selected from the group consisting of a condensed aromatic ring, a substituted condensed aromatic ring, carboxyl, alkoxycarbonyl, and

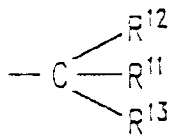

wherein $R^{11}$ is H or OH, or $R^{11}$ can form a bond with $R^2$ when $Z^1$ is C and $n^1$ is 0; $R^{12}$ and $R^{13}$ are independently phenyl, substituted phenyl, a heterocyclic ring, or a substituted heterocyclic ring, or $R^{12}$ and $R^{13}$ can form a condensed ring; $R^2$ is H when forming no bond with $R^3$ or $R^{11}$; $R^3$ is H when forming no bond with $R^2$; $k^1$ is an integer of 2 to 5; $Y^1$ is a group selected from the group consisting of O, S, SO, $SO_2$, $CH_2$, and

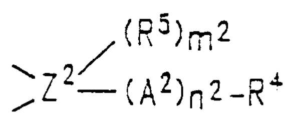

wherein $Z^2$ is N or C; $m^2$ is 0 when $Z^2$ is N and $m^2$ is 1 when $Z^2$ is C; $A^2$ is O, $SO_2$, or $CH_2$; $n^2$ is 0 or 1 when $A^2$ is O or $SO_2$; $n^2$ is an integer of 0 to 3 when $A^2$ is $CH_2$; $R^4$ is a group selected from the group consisting of alkyl, phenyl, substituted phenyl, a heterocyclic ring, a substituted heterocyclic ring, $-CO-R^{41}$, and

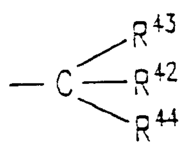

wherein $R^{41}$ is a group selected from the group consisting of OH, alkoxy, amino, arylalkyloxy, substituted amino, arylalkenyl, and substituted arylalkenyl; $R^{42}$ is H or OH, or $R^{42}$ can form a bond with $R^5$ when $Z^2$ is C and $n^2$ is 0; $R^{43}$ and $R^{44}$ are independently phenyl, substituted phenyl, a heterocyclic ring, or a substituted heterocyclic ring; $R^5$ is H when forming no bond with $R^{42}$; $Y^2$ is $CH_2$ or CO; $Y^3$ is $(CH_2)_2$ or phenylene; and $k^2$ is 0 or 1,
or pharmaceutically acceptable salts thereof.

2. The compound according to claim 1 represented by the following Formula I-1:

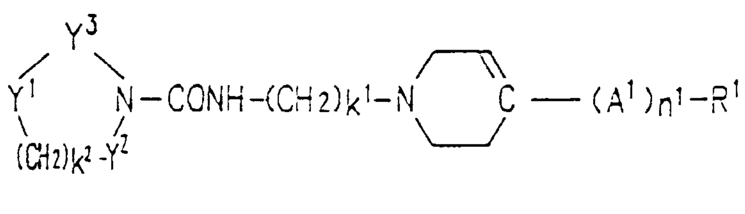

(I-1)

wherein $A^1$, $R^1$, $Y^1$, $Y^2$, $Y^3$, $k^1$, $k^2$, and $n^1$ are the same as defined in claim 1.

3. The compound according to claim 1, wherein $R^1$ is quinolyl or

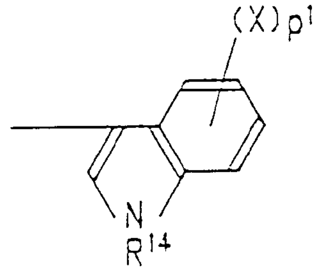

wherein $R^{14}$ is H, phenyl, or substituted phenyl; $p^1$ is an integer of 0 to 4; and X's are independently a group selected from the group consisting of Cl, F, $CH_3$, CN, and $CH_2COOH$.

4. The compound according to claim 1 represented by the following Formula I-2:

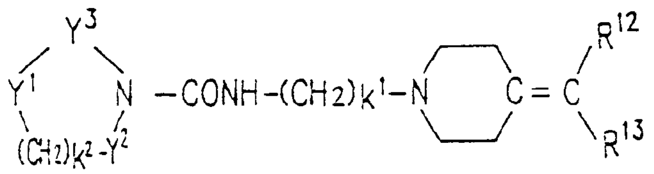

(I-2)

wherein $R^{12}$, $R^{13}$, $Y^1$, $Y^2$, $Y^3$, $k^1$, and $k^2$ are the same as defined in claim 1.

5. The compound according to claim 1, wherein $R^1$ is

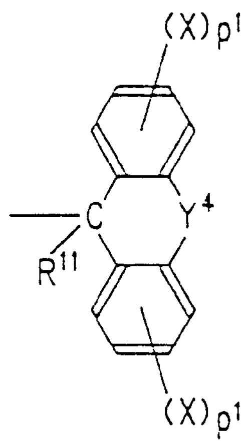

wherein $Y^4$ is $(CH_2)k^3$ or $CH_2$-$Y^5$, where $k^3$ is an integer of 0 to 2, $Y^5$ is O, S, or $NR^5$, where $R^5$ is H or $CH_3$; $p^1$ is an integer of 0 to 4; and X's are independently a group selected from the group consisting of Cl, F, $CH_3$, CN, and $CH_2COOH$.

6. The compound according to claim 1, wherein $R^4$ is a group selected from the group consisting of pyridyl, pyrimidinyl, imidazolyl, quinolyl, and

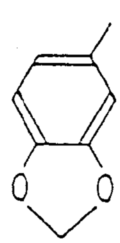

7. The compound according to claim 1 represented by the following Formula I-3:

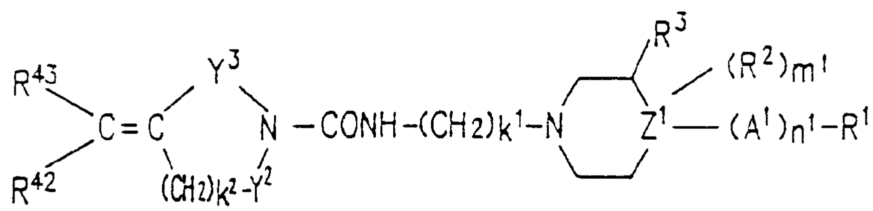 (I-3)

wherein $A^1$, $R^1$, $R^2$, $R^3$, $R^{43}$, $R^{42}$, $Y^2$, $Y^3$, $Z^1$, $k^1$, $k^2$, $m^1$, and $n^1$ are the same as defined in claim 1.

8. The compound according to claim 1 represented by the following Formula I-4:

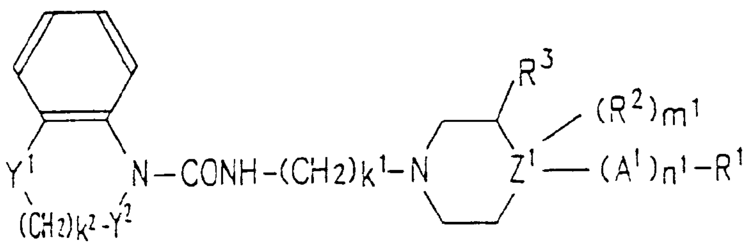 (I-4)

wherein $A^1$, $R^1$, $R^2$, $R^3$, $Y^1$, $Y^2$, $Z^1$, $k^1$, $k^2$, $m^1$, and $n^1$ are the same as defined in claim 1.

9. The compound according to claim 1, wherein $A^1$ is $CH_2$ and $R^1$ is carboxyl or alkoxycarbonyl.

10. The compound according to claim 1, wherein $Z^1$ is N and $n^1$ is 0.

11. The compound according to claim 1, wherein $Z^1$ is C, $A^1$ is O, an $n^1$ is 1.

12. the compound according to claim 1, wherein $R^1$ is represented by the following Formula:

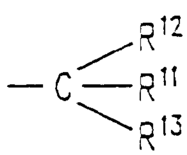

wherein $R^{11}$, $R^{12}$, and $R^{13}$ are the same as defined in claim 1.

13. The compound according to claim 12, wherein $R^{12}$ and $R^{13}$ are phenyl or substituted phenyl.

14. The compound according to claim 12, wherein at least one of $R^{12}$ and $R^{13}$ is pyridyl.

15. The compound according to claim 1, wherein $Y^3$ is $(CH_2)_2$.

16. The compound according to claim 15, wherein $Y^2$ is $CH_2$ and $k^2$ is 1.

17. A compound according to claim 15, wherein $Y^1$ is S or $CH_2$, and $Y^2$ is CO.

18. An antiallergic agent containing an effective amount of a compound according to claim 1 or pharmaceutically acceptable salts thereof.

19. A composition comprising the compound according to claim 1 or pharmaceutically acceptable salts thereof and a carrier.

European Patent Office

# EUROPEAN SEARCH REPORT

Application Number
EP 94 30 4289

## DOCUMENTS CONSIDERED TO BE RELEVANT

| Category | Citation of document with indication, where appropriate, of relevant passages | Relevant to claim |
|---|---|---|
| D,A | EP-A-0 224 919 (FUJISAWA) 10 June 1987<br>* page 1 - page 2, line 26 *<br>& JP-A-63 225 374 (...)<br>--- | 1-19 |
| P,A | EP-A-0 580 398 (SHIONOGI) 26 January 1994<br>* the whole document *<br>--- | 1-19 |
| P,A | EP-A-0 597 112 (KYOTO) 18 May 1994<br>* page 1 - page 2, line 21 *<br>--- | 1-19 |
| X | EP-A-0 526 434 (BOEHRINGER INGELHEIM) 3 February 1993<br>* claim 1 *<br>--- | 1,8,10, 12-14,19 |
| X | CHEMICAL ABSTRACTS, vol. 114, no. 19, 13 May 1991, Columbus, Ohio, US;<br>abstract no. 185435,<br>E. ZARA-KACZIAN ET. AL. 'Synthesis of 1-aryl-1,4-dihydro-3(2H)-isoquinolines with piperazine ring in the side chain having potential antiserotoninergic activity.'<br>* abstract *<br>& ACTA CHIM. HUNG.,<br>vol.127, no.4, 1990<br>pages 607 - 627<br>--- | 1 |
| A | JOURNAL OF MEDICINAL CHEMISTRY.,<br>vol.33, no.7, July 1990, WASHINGTON US<br>pages 2028 - 2032<br>D. A. WALSH ET. AL. 'Synthesis and antiallergic Activity of N-[2-(Dimethylamino)ethyl]-4-aryl-1-piperazinecarboxamide Derivatives.'<br>* the whole document *<br>---<br>-/-- | 1-19 |

CLASSIFICATION OF THE APPLICATION (Int.Cl.5)

C07D295/20
C07D295/22
C07D207/26
C07D211/14
C07D211/22
C07D211/46
A61K31/445
A61K31/50
A61K31/54
C07D211/70
C07D213/74
C07D239/42
C07D241/20
C07D279/12
C07D401/04
C07D401/12
C07D417/12
C07D417/14

TECHNICAL FIELDS SEARCHED (Int.Cl.5)

C07D

The present search report has been drawn up for all claims

| Place of search | Date of completion of the search | Examiner |
|---|---|---|
| THE HAGUE | 27 September 1994 | Kissler, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 30 4289

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | EP-A-0 129 207 (BOEHRINGER INGELHEIM) 27 December 1984<br>* the whole document *<br>--- | 1-19 | |
| D,A | EP-A-0 277 725 (ROBINS) 10 August 1988<br>* page 1 - page 2, line 55 *<br>& JP-A-63 188 670 (...)<br>--- | 1-19 | |
| A | EP-A-0 373 226 (TAIHO) 20 June 1990<br>* page 2, line 17 - page 6, line 2 *<br>--- | 1-19 | |
| A | EP-A-0 304 330 (SHIONOGI) 22 February 1989<br>* the whole document *<br>--- | 1-19 | |
| A | EP-A-0 309 043 (JANSSEN) 29 March 1989<br>* abstract *<br>--- | 1-19 | |
| A | EP-A-0 445 701 (SHIONOGI) 11 September 1991<br>* the whole document *<br>----- | 1-19 | |
| | | | TECHNICAL FIELDS SEARCHED (Int.Cl.5) |

The present search report has been drawn up for all claims

| Place of search | Date of completion of the search | Examiner |
|---|---|---|
| THE HAGUE | 27 September 1994 | Kissler, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)